# EUROPEAN PATENT APPLICATION

(11) **EP 4 052 742 A2**
(43) Date of publication of application: **07.09.2022**
(21) Application number: 20883467.1
(22) Date of filing: 29.10.2020
(51) Int. Cl.: A61M 5/168, A61M 5/142, A61M 5/14, A61M 5/165, A61M 5/36

(54) **MEDICINAL LIQUID SUPPLY CONTROL DEVICE, RESERVOIR ASSEMBLY FOR MEDICINAL LIQUID SUPPLY CONTROL DEVICE, AND MEDICINAL LIQUID INJECTION APPARATUS COMPRISING SAME**

(30) Priority: 01.11.2019 KR 20190138895; 13.01.2020 KR 20200004336; 13.01.2020 KR 20200004355
(71) Applicant: Kim, Yong Hyun, Goyang-si, Gyeonggi-do 10483 (KR)
(72) Inventor: Kim, Yong Hyun, Goyang-si, Gyeonggi-do 10483 (KR)
(74) Representative: Santarelli
(86) International application number: PCT/KR2020/014896
(87) International publication number: WO 2021/086043

(57) **Abstract**

A medicinal liquid supply control apparatus according to an embodiment of the present disclosure includes a reservoir located on a second channel to define an internal space that stores a medicinal liquid, an inlet hole and an outlet hole being formed in one portion of an outer surface of the reservoir and connected to the internal space, and a reservoir support part on which the one portion of the reservoir is fixed, an inlet connection hole connected to the inlet hole and an outlet connection hole connected to the outlet hole being formed at the reservoir support part.

## Description

### TECHNICAL FIELD

The present disclosure relates to a medicinal liquid supply control apparatus for controlling a supply amount of a medicinal liquid, a reservoir assembly for the medicinal liquid supply control apparatus, and a medicinal liquid injection apparatus including the medicinal liquid supply control apparatus.

### BACKGROUND

In order to supply a drug to a patient, a medicinal liquid injection apparatus for injecting a medicinal liquid of a liquid state (e.g., an injection solution) to a patient is known. Using the medicinal liquid injection apparatus, the medicinal liquid in a predetermined storage space is introduced into the body of the patient through a passage (e.g., the inner spaces of a tube and an injection needle) connected to a patient.

There is known a priming operation for filling the inside of a channel, in which the medical liquid is to move, with a priming liquid (e.g., a medical liquid or a saline solution to be injected) before a member to be connected to the patient, such as a catheter or an injection needle, is coupled in order to prevent air from flowing into the patient's body during the use of the medical liquid injection apparatus.

Meanwhile, among medicinal liquids, there are medicinal liquids (e.g., antibiotics, analgesics, and anticancer drugs) that should be injected into a patient's body in a manner of limiting an injection dose per hour. In principle, such medicinal liquids are injected into the patient's body at a constant dose per hour. However, depending on conditions such as a patient's condition, there is also a need to temporarily increase the supply dose of a medicinal liquid to be injected. This injection performed in the state of temporarily increasing a medicinal liquid is called a bolus injection. There is known a medicinal liquid supply control apparatus for controlling an injection dose of a medicinal liquid per hour for a bolus injection.

### SUMMARY

Embodiments of the present disclosure provide an apparatus capable of reducing a remaining amount of a medicinal liquid when injecting the medicinal liquid into a patient's body.

Embodiments of the present disclosure provide an apparatus capable of reducing a time required to fill a priming liquid in a channel of a medicinal liquid injection apparatus.

An aspect of the present disclosure provides embodiments of a medicinal liquid supply control apparatus having a medicinal liquid channel that guides the flow of a medicinal liquid. The medicinal liquid channel of the medicinal liquid supply control apparatus according to a representative embodiment includes: a first channel that guides the medicinal liquid to flow from an inlet to an outlet; and a second channel that guides the medicinal liquid to split at a branching point of the first channel, guides the split medicinal liquid to merge at a merging point located downstream of the branching point of the first channel, and is configured to be capable of opening and closing. The medicinal liquid supply control apparatus includes: a reservoir located on the second channel to define an internal space that stores the medicinal liquid, an inlet hole and an outlet hole being formed in one portion of an outer surface of the reservoir and connected to the internal space; and a reservoir support part on which the one portion of the reservoir is fixed, an inlet connection hole constituting a portion of the second channel is connected to the inlet hole, and an outlet connection hole constituting a portion of the second channel is connected to the outlet hole being formed at the reservoir support part.

In embodiments, the reservoir support part may be disposed below the reservoir. The medicinal liquid supply control apparatus may further include a reservoir pushing member disposed at an upper side of the reservoir and configured to be capable of pressing the reservoir by moving downward.

In embodiments, a gap in an upper-lower direction between an upper surface of the reservoir support part and a lower surface of the reservoir pushing member at a position corresponding to the one portion may be larger than a gap in the upper-lower direction between the upper surface and the lower surface at a position different from the position corresponding to the one portion.

In embodiments, one of an upper surface of the reservoir support part and a lower surface of the reservoir pushing member may include a convex surface toward the other one, and the other one may include a concave surface corresponding to the convex surface.

In embodiments, the one portion may be located at a center of a lower surface of the reservoir. A gap in an upper-lower direction between the convex surface and the concave surface in a central portion of the reservoir may be larger than a gap in the upper-lower direction between the convex surface and the concave surface in an edge portion of the reservoir.

In embodiments, the one portion may be located at a center of a lower surface of the reservoir. On a cross section crossing a center of the reservoir upward and downward, the concave surface may have a larger curvature than the convex surface.

In embodiments, the lower surface of the reservoir pushing member may be formed of a material that is more flexible than the reservoir support part.

In embodiments, the reservoir pushing member may include a reservoir pressing portion having a lower surface that presses the reservoir, and an upper plate coupled to the upper side of the reservoir pressing portion. The reservoir pressing portion may be formed of a material that is more flexible than the upper plate.

In embodiments, the medicinal liquid supply control apparatus may further include: a connection part having an upper side to which the reservoir support part is coupled; and a sealing plate sandwiched between the reservoir support part and the connection part to be in contact with the reservoir support part and the connection part, a first connection hole that constitutes a portion of the second channel and is connected to the inlet connection hole, and a second connection hole that constitutes a portion of the second channel and is connected to the outlet connection hole being formed at the sealing plate.

In embodiments, the reservoir may be configured to change a volume of the internal space.

Another aspect of the present disclosure provides embodiments of a reservoir assembly for a medicinal liquid supply control apparatus. A reservoir assembly according to a representative element may include: a reservoir located on the medicinal liquid channel to define an internal space that stores the medicinal liquid, an inlet hole and an outlet hole being formed in one portion of an outer surface of the reservoir and connected to the internal space; and a reservoir support part on which the one portion of the reservoir is fixed, an inlet connection hole constituting a portion of the medicinal liquid channel is connected to the inlet hole, and an outlet connection hole constituting a portion of the medicinal liquid channel is connected to the outlet hole being formed at the reservoir support part.

In embodiments, an upper surface of the reservoir support part may include an upwardly convex surface.

In embodiments, the one portion may be located at a center of a lower surface of the reservoir, and the convex surface may be formed to protrude from a position corresponding to the center.

In embodiments, the inlet hole and the inlet connection hole may be vertically connected to each other, and the outlet hole and the outlet connection hole may be vertically connected to each other.

Another aspect of the present disclosure provides embodiments of a medicinal liquid injection apparatus. A medicinal liquid injection apparatus according to a representative embodiment includes: a pumping module configured to press a medicinal liquid; an extension tube configured to allow the medicinal liquid flowing out from the pumping module by being pressed in the pumping module to flow therethrough; and a medicinal liquid supply control apparatus having a medicinal liquid channel connected to the extension tube. The medicinal liquid channel includes: a first channel that guides the medicinal liquid to flow from an inlet to an outlet; and a second channel that guides the medicinal liquid to split at a branching point of the first channel, guides the split medicinal liquid to merge at a merging point located downstream of the branching point of the first channel, and is configured to be capable of opening and closing. The medicinal liquid supply control apparatus includes: a reservoir located on the second channel to define an internal space that stores the medicinal liquid, an inlet hole and an outlet hole being formed in one portion of an outer surface of the reservoir and connected to the internal space; and a reservoir support part on which the one portion of the reservoir is fixed, an inlet connection hole constituting a portion of the second channel is connected to the inlet hole, and an outlet connection hole constituting a portion of the second channel is connected to the outlet hole being formed at the reservoir support part.

The medicinal liquid channel of the medicinal liquid supply control apparatus according to another representative embodiment includes: a first channel that guides the medicinal liquid to flow from an inlet to an outlet; and a second channel that guides the medicinal liquid to split at a branching point of the first channel, guides the split medicinal liquid to merge at a merging point located downstream of the branching point of the first channel, and is configured to be capable of opening and closing. The medicinal liquid supply control apparatus may include: a body including a plurality of parts assembled with each other; and at least one sealing plate sandwiched between two adjacent parts of the plurality of parts and being in contact with the two adjacent parts. One of the two adjacent parts is recessed in a direction opposite to the direction in contact with the surface of the sealing plate to define at least one channel groove extending along the surface, wherein the channel groove is covered by the sealing plate and constitutes a portion of the medicinal liquid channel.

In embodiments, the medicinal liquid channel may penetrate the two adjacent parts and the sealing plate.

In embodiments, a connection hole that is connected to the channel groove and constitutes a portion of the medicinal liquid channel may be formed at the sealing plate.

In embodiments, a portion of the medicinal liquid channel may be formed at the other one of the two adjacent parts, and the portion of the medicinal liquid channel of the other one, the connection hole, and the channel groove may be sequentially connected to each other.

In embodiments, at least one extension hole that is connected to the channel groove and constitutes a portion of the medicinal liquid channel may be formed at one of the two adjacent parts.

In embodiments, the at least one extension hole may include a plurality of extension holes.

In embodiments, the one of the two adjacent parts may include a channel guide rib protruding toward the surface, extending along the surface to partition the channel groove, and being in contact with the sealing plate.

In embodiments, the material of the sealing plate may be more flexible than that of the two adjacent parts.

In embodiments, a first split-flow guide point may be located between the branching point and the merging point on the first channel, and a second split-flow guide point may be located between the branching point and the merging point on the second channel. The branching point, the first split-flow guide point, and the second split-flow guide point may be located in the channel groove.

In embodiments, a first merging guide point may be located between the branching point and the merging point on the first channel, and a second merging guide point may be located between the branching point and the merging point on the second channel. The merging point, the first merging guide point, and the second merging guide point may be located in the channel groove.

In embodiments, the at least one channel groove may include a branching channel groove and a merging channel groove. A first split-flow guide point may be located between the branching point and the merging point on the first channel, and a second split-flow guide point may be located between the branching point and the merging point on the second channel. The branching point, the first split-flow guide point, and the second split-flow guide point may be located in the branching channel groove. A first merging guide point may be located between the first split-flow guide point and the merging point on the first channel, and a second merging guide point may be located between the second split-flow guide point and the merging point on the second channel. The merging point, the first merging guide point, and the second merging guide point may be located in the merging channel groove.

In embodiments, the inlet and the outlet may be formed at the other one of the two adjacent parts.

In embodiments, the medicinal liquid supply control apparatus may further include a reservoir that is located on the second channel and defines an internal space for storing a medicinal liquid. On the second channel, a reservoir inlet channel may be located between the branching point and the internal space, and a reservoir outlet channel may be located between the merging point and the internal space. The at least one channel groove may include: an inlet channel groove that is covered by the sealing plate and constitutes a portion of the reservoir inlet channel; and an outlet channel groove that is covered by the sealing plate and constitutes a portion of the reservoir outlet channel.

In embodiments, on the first channel, a first connection guide channel may be located between the branching point and the merging point. The at least one channel groove may further include a connection channel groove that is covered by the sealing plate and constitutes the first connection guide channel.

In embodiments, on the first channel, a first connection guide channel may be located between the branching point and the merging point. The channel groove may be covered by the sealing plate and constitute the first connection guide channel.

In embodiments, the medicinal liquid supply control apparatus may further include a reservoir that is located on the second channel and defines an internal space for storing a medicinal liquid. The reservoir may be supported on the other one of the two adjacent parts.

In embodiments, the medicinal liquid supply control apparatus may further include: a first medicinal liquid transfer tube that defines a capillary channel constituting a portion of the first channel and is disposed in at least one of the plurality of parts; and a second medicinal liquid transfer tube that defines a capillary channel constituting a portion of the second channel and is disposed in at least one of the plurality of parts.

In embodiments, the plurality of parts may include: a second part that supports one end of the first medicinal liquid transfer tube and one end of the second medicinal liquid transfer tube; and a third part that is coupled to the second part and supports the other end of the first medicinal liquid transfer tube and the other end of the second medicinal liquid transfer tube.

In embodiments, the plurality of parts may include a first part and a second part that are coupled to each other and a third part and a fourth part that are coupled to each other. The at least one sealing plate may include: a lower sealing plate sandwiched between the first part and the second part; and an upper sealing plate sandwiched between the third part and the fourth part.

In embodiments, the medicinal liquid supply control apparatus may further include: an upstream air passage filter located upstream of the branching point on the first channel and configured to discharge the air in the first channel to the outside; and a downstream air passage filter located downstream of the merging point on the first channel and configured to discharge the air in the first channel to the outside.

A medicinal liquid injection apparatus according to another embodiment of the present disclosure includes: a pumping module configured to press a medicinal liquid; an extension tube configured to allow the medicinal liquid flowing out from the pumping module by being pressed in the pumping module to flow through the extension tube; and a medicinal liquid supply control apparatus having a medicinal liquid channel connected to the extension tube. The medicinal liquid channel includes: a first channel that guides the medicinal liquid to flow from an inlet to an outlet; and a second channel that guides the medicinal liquid to split at a branching point of the first channel, guides the split medicinal liquid to merge at a merging point located downstream of the branching point of the first channel, and is configured to be capable of opening and closing. The medicinal liquid supply control apparatus may include: a body including a plurality of parts assembled with each other; and at least one sealing plate sandwiched between two adjacent parts of the plurality of parts and being in contact with the two adjacent parts. One of the two adjacent parts is recessed in a direction opposite to the direction in contact with the surface of the sealing plate to define at least one channel groove extending along the surface. The channel groove is covered by the sealing plate and constitutes a portion of the medicinal liquid channel.

According to the embodiments of the present disclosure, by reducing or eliminating the use of a tube in forming a medicinal liquid channel required for a medicinal liquid supply control apparatus, it is possible to reduce the remaining amount of the lost medicinal liquid and to reduce a time required for filling a priming liquid.

According to the embodiments of the present disclosure, since it is possible to configure a medicinal liquid channel of the medicinal liquid only by coupling components other than a tube, it is possible to improve the manufacturing convenience of a medicinal liquid supply control apparatus.

### BRIEF DESCRIPTION OF THE DRAWINGS

FIG. 1 is a conceptual view illustrating the entire system of a medicinal liquid injection apparatus 1 according to an embodiment of the present disclosure.
FIG. 2 is a perspective view of a medicinal liquid supply control apparatus 80 according to a first embodiment of the present disclosure, illustrating a figure in which a user operates a button member 700 in a state in which a cotter 1100 is removed.
FIG. 3 is a perspective view illustrating a state in which a case 100 is removed from the medicinal liquid supply control apparatus 80 of FIG. 2.
FIG. 4 is a perspective view illustrating a state in which the button member 700 is removed from the medicinal liquid supply control apparatus 80 of FIG. 3.
FIG. 5 is an exploded perspective view of the medicinal liquid supply control apparatus 80 of FIG. 1.
FIG. 6 is an exploded perspective view of the medicinal liquid supply control apparatus 80 of FIG. 5 viewed from another angle.
FIG. 7 is an exploded perspective view illustrating only some of the components of FIG. 5, in which unlike FIG. 5, some components are illustrated in a state of being assembled with each other.
FIG. 8 is an exploded perspective view illustrating the state of FIG. 7 viewed from another angle, in which an enlarged view (E) of a portion is illustrated.
FIG. 9 is an elevation view of a reservoir assembly A400 of FIG. 7.
FIG. 10 is a perspective view illustrating a cross section of the medicinal liquid supply control apparatus 80 taken along line S1-S1' in FIG. 8.
FIG. 11 is a cross-sectional view of the medicinal liquid supply control apparatus 80 taken along line S2-S2' in FIG. 8.
FIG. 12 is a cross-sectional view of the medicinal liquid supply control apparatus 80 taken along line S3-S3' in FIG. 8.
FIG. 13 is a cross-sectional view of the medicinal liquid supply control apparatus 80 taken along line S4-S4' in FIG. 8.
FIG. 14 is an exploded perspective view of a lock assembly 600 and a body 200 of FIG. 5.
FIG. 15 is a perspective view illustrating a state in which the lock assembly 600 of FIG. 14 is coupled.
FIG. 16 is a perspective view of the button member 700 of FIG. 5 in a state in which a portion thereof is cut away.
FIG. 17A is a cross-sectional views of the medicinal liquid supply control apparatus 80 taken along line S5-S5' in FIG. 8, illustrating a locked state.
FIG. 17B is a cross-sectional view of the medicinal liquid supply control apparatus 80 taken along line S5-S5' in FIG. 8, illustrating a released state.
FIG. 18 is a perspective view of a medicinal liquid supply control apparatus 80' according to a second embodiment of the present disclosure, illustrating ways in which cotters 1100 can be coupled to the medicinal liquid supply control apparatus 80'.
FIG. 19 is a perspective view illustrating a state in which the case 100 is removed from the medicinal liquid supply control apparatus 80' of FIG. 18.
FIG. 20 is a perspective view illustrating a state in which an inner housing 1500 is additionally removed from the medicinal liquid supply control apparatus 80' of FIG. 19.
FIG. 21 is a perspective view illustrating a state in which the button member 700, a selector 1800, a lock bar 1900, and the like are additionally removed from the medicinal liquid supply control apparatus 80' of FIG. 20.
FIG. 22 is an exploded perspective view of the medicinal liquid supply control apparatus 80' of FIG. 18.
FIG. 23 is an exploded perspective view of the medicinal liquid supply control apparatus 80' of FIG. 22 viewed from another angle.
FIG. 24 is an exploded perspective view illustrating only some of the components of FIG. 22, in which unlike FIG. 22, some components are illustrated in the state of being assembled with each other.
FIG. 25 is an exploded perspective view viewed from another angle of FIG. 24, in which an enlarged view (E) of a portion is illustrated.
FIG. 26 is a perspective view illustrating a cross section of the medicinal liquid supply control apparatus 80' taken along line S6-S6' in FIG. 34.
FIG. 27 is a cross-sectional view of the medicinal liquid supply control apparatus 80' taken along line S7-S7' in FIG. 25.
FIG. 28 is a cross-sectional view of the medicinal liquid supply control apparatus 80' taken along line S8-S8' in FIG. 25.
FIG. 29 is a cross-sectional view of the medicinal liquid supply control apparatus 80' taken along line S9-S9' in FIG. 25.
FIG. 30 is a cross-sectional view of the medicinal liquid supply control apparatus 80' taken along line S10-S10' in FIG. 25.

### DETAILED DESCRIPTION

Embodiments of the present disclosure are illustrated for the purpose of explaining the technical idea of the present disclosure. The scope of the rights according to the present disclosure is not limited to the embodiments presented below or the detailed descriptions of such embodiments.

All technical and scientific terms used in the present disclosure have the meaning generally understood by those of ordinary skill in the art to which the present disclosure pertains, unless otherwise defined. All terms used in the present disclosure are chosen for the purpose of more clearly describing the present disclosure and are not chosen to limit the scope of rights according to the present disclosure.

As used in the present disclosure, expressions such as "comprising", "including", "having", and the like are to be understood as open-ended terms having the possibility of encompassing other embodiments, unless otherwise mentioned in the phrase or sentence containing such expressions.

The singular form described in the present disclosure may include a plural meaning, unless otherwise mentioned. This applies equally to the singular form recited in the claims.

The terms "first", "second", and the like used in the present disclosure are used to distinguish a plurality of components from one another, and do not limit the order or importance of the corresponding components.

In the present disclosure, where it is mentioned in the present disclosure that one element is "connected" or "coupled" to another element, it is to be understood that said one element may be directly connected or coupled to another element, or may be connected or coupled to said another element via a new additional element.

Direction indicating words such as "upward", "upper", and the like used in the present disclosure mean a direction of arrow U in the accompanying drawings, and direction indicating words such as "downward", "lower", and the like mean a direction of arrow D in the accompanying drawings, but these are merely references for explaining the present disclosure such that the present disclosure can be clearly understood, and depending on what is the reference, the direction indicating words may be interpreted accordingly.

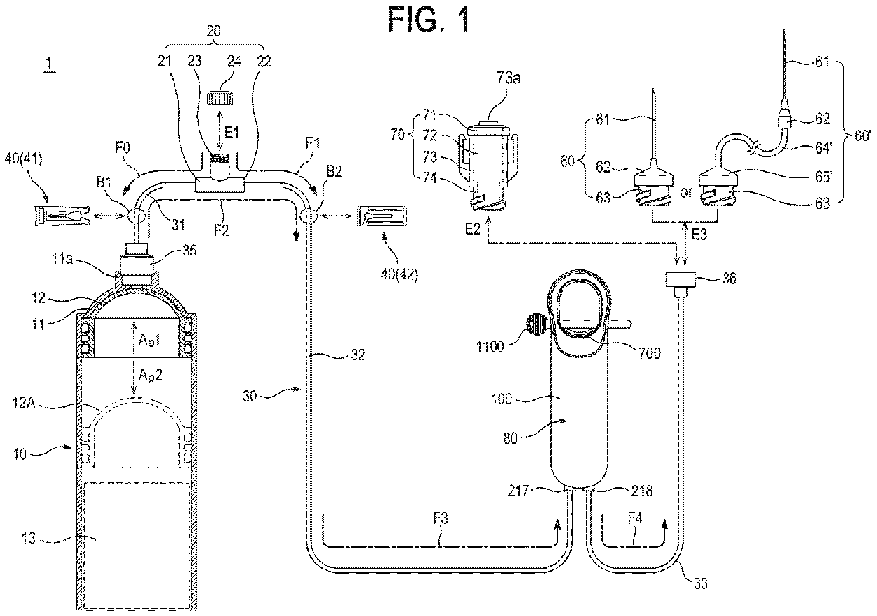

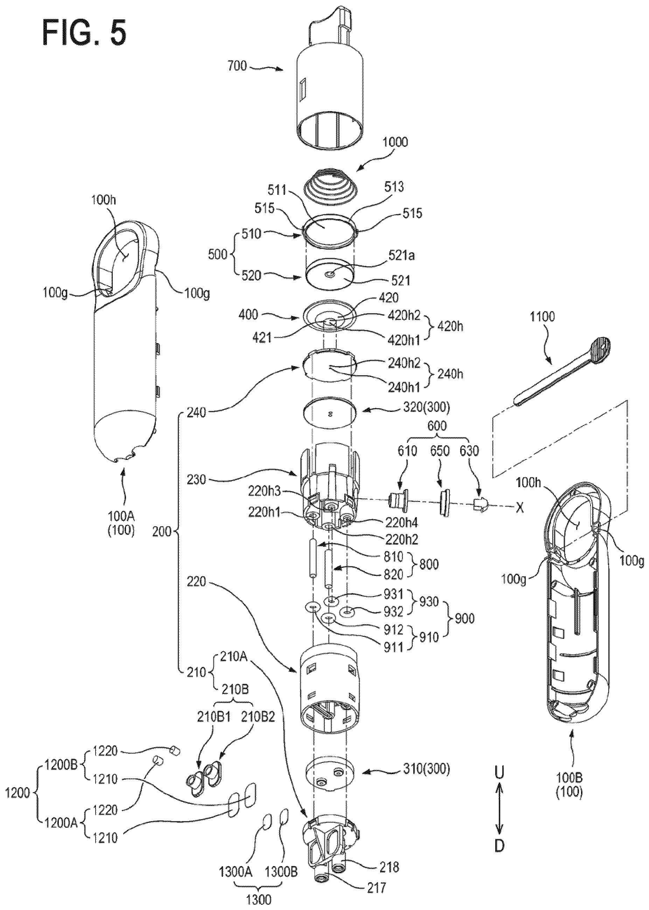

"Upstream" and "downstream" used in the present disclosure are defined based on a direction in which a medicinal liquid flows when a pumping module 10 presses the medicinal liquid. Specifically, directions of arrows F2 and F3 of FIG. 1 and arrow F4 of FIG. 5 are defined as a downstream direction, and a direction opposite to the downstream direction is defined as an upstream direction.

A "medicinal liquid" used in the present disclosure should be understood to include not only a liquid containing a therapeutic substance, but also a liquid that assists the therapeutic substance or can be used together with the therapeutic substance, and a liquid that can be injected into a patient. A priming liquid, which will be described later, is one of these medicinal liquids.

Hereinafter, embodiments of the present disclosure will be described with reference to the accompanying drawings. In the accompanying drawings, identical or corresponding components may be indicated by identical reference numerals. In the following description of embodiments, repeated descriptions of the identical or relevant components will be omitted. However, even if a description of a component is omitted, such a component is not intended to be excluded in an embodiment.

FIG. 1 is a conceptual view illustrating the entire system of a medicinal liquid injection apparatus 1 according to an embodiment of the present disclosure. The process of injecting a medicinal liquid into a patient using the medicinal liquid injection apparatus 1 according to embodiments of the present disclosure includes a priming step and a medicinal liquid injection step, which proceed sequentially.

Referring to FIG. 1, in the priming step, a priming liquid flows along an extension tube 30. The priming liquid flowing along the extension tube 30 is introduced into the medicinal liquid supply control apparatus 80. The medicinal liquid injection apparatus 1 may include an end cap 70 detachably connected to the downstream side of the medicinal liquid supply control apparatus 80. Air inside the extension tube 30 may be discharged to the outside through the end cap 70.

Accordingly, the interiors of the extension tube 30 and the medicinal liquid supply control apparatus 80 are filed with the priming liquid. The priming liquid may be a liquid containing a therapeutic substance or a liquid that can be injected into a patient, such as a saline solution.

The end cap 70 is configured such that the air passing through the medicinal liquid supply control apparatus 80 and the priming liquid are introduced into the end cap 70. The end cap 70 may be configured to allow air to flow out to the outside, but to prevent the priming liquid from flowing out to the outside.

The end cap 70 includes a vent filter 71 that blocks the passage of the priming liquid therethrough but allows the passage of gas therethrough. The vent filter 71 includes a hydrophobic filter. The end cap 70 may include a sponge 72 disposed upstream of the vent filter 71. The end cap 70 includes an end cap casing 73 accommodating the vent filter 71 therein. The end cap casing 73 accommodates the sponge 72 therein. The end cap casing 73 forms a vent hole 73a through which the gas passes. The end cap 70 includes an end cap coupling portion 74 configured to be coupled with a downstream connection portion 36 constituting the downstream end of the extension tube 30 of the medicinal liquid injection apparatus 1. Arrow E2 in FIG. 1 illustrates the coupling and decoupling direction of the end cap coupling portion 74 with respect to the downstream connection portion 36.

When the end cap 70 is filled with the priming liquid, the end cap 70 may be separated from the medicinal liquid supply control apparatus 80, and the medicinal liquid supply control apparatus 80 and a patient connection unit 60 or 60' may be connected to each other.

The patient connection unit 60 or 60' may include an injection needle 61, a catheter, or the like. The patient connection unit 60 or 60' includes a component that is introduced into a patient's body, such as the injection needle 61.

The patient connection unit 60 or 60' may include "an introduction component including a component to be introduced into the patient's body, such as the injection needle 61" and "a remaining component". The introduction component and the remaining component may be detachably coupled to each other. In this case, in the state in which the introduction component is connected to the patient but is separated from the remaining component, the user may couple the remaining component to the medicinal liquid supply control apparatus 80 and may then couple the introduction component and the remaining component to each other. In this case, the liquid passing through the medicinal liquid supply control apparatus 80 may be introduced into the patient's body after sequentially passing through the remaining component and the introduction component.

The patient connection unit 60 or 60' includes an injection support portion 62 that supports the injection needle 61. The patient connection unit 60 or 60' includes a unit coupling portion 63 configured to be coupled with the downstream connection portion 36 of the medicinal liquid injection apparatus 1. Arrow E3 in FIG. 1 illustrates the direction of coupling and decoupling the unit coupling portion 63 with respect to the downstream connection portion 36.

As an example, the patient connection unit 60 may be configured such that the injection needle 61, the injection support portion 62, and the unit coupling portion 63 are sequentially connected.

As another example, the patient connection unit 60' further includes a patient connection tube fixing portion 65' connected to the downstream side of the unit coupling portion 63. The patient connection unit 60' further includes a patient connection tube 64' that interconnects the patient connection tube fixing portion 65' and the injection support portion 62. The patient connection tube 64' may be formed of a flexible material. The patient connection unit 60' may be configured by sequentially connecting the injection needle 61, the injection support portion 62, the patient connection tube 64', the patient connection tube fixing portion 65', and the unit coupling portion 63.

In the medicinal liquid injection step, the medicinal liquid is introduced into the patient's body in response to the pressing in the pumping module 10. The medicinal liquid in the medicinal liquid injection step is a liquid containing a therapeutic substance. When the priming liquid is not a liquid containing a therapeutic substance but a saline solution, the priming liquid first flows into the patient's body, and the liquid containing the therapeutic substance that flows following the priming liquid may be introduced into the patient's body.

The pumping module 10 includes a chamber 11 configured to accommodate a medicinal liquid. The chamber 11 defines an internal space with a pressing unit 12. The medical liquid may be stored in the inner space. In another embodiment, a saline solution or the like may be temporarily stored in the inner space. In the chamber 11, a discharge port portion 11a through which the liquid inside the chamber 11 is discharged is formed.

The pumping module 10 is configured to press a medicinal liquid. The pumping module 10 includes a pressing unit 12 configured to pressurize the liquid within the chamber 11. The pressing unit 12 may pressurize the liquid in the chamber 11 by moving in a predetermined pressurizing direction Ap 1. When the inside of the chamber 11 is being filled with the liquid, the pressing unit 12 moves in the direction Ap2 opposite the pressurizing direction Ap1. FIG. 1 illustrates a position in the state in which the pressing unit 12 moves in the opposite direction Ap2 with reference to 12A.

The pumping module 10 may include a pressing operation portion 13 that provides power to move the pressing unit 12 in a pressing direction Ap1. As an example, the pressing operation portion 13 may be configured to press the liquid in the chamber 11 by using a volumetric expansion by gas activation. As another example, the pressing operation portion 13 may provide a portion that can be held by the user, and may move the pressing unit 12 in the pressing direction Ap1 with the force of the user.

Although not illustrated, as another example, the pressing unit 12 may be configured to press the liquid by using the elastic force of an elastic body such as a balloon. In this case, the pressing unit 12 may be configured to press the liquid in the balloon.

The medicinal liquid injection valve 20 is configured to be capable of filling the chamber 11 with the liquid. The liquid may be introduced into the extension tube 30 or the chamber 11 through the medicinal liquid injection valve 20 from the outside. The medicinal liquid injection valve 20 is connected to the extension tube 30, but in another embodiment (not illustrated), the medicinal liquid injection valve 20 may be connected to the chamber 11.

The medicinal liquid injection valve 20 includes a first extension portion 21 connected to the downstream end of a first connection portion 31 of the extension tube 30, and a second extension portion 22 connected to the upstream end of the second connection portion 32 of the extension tube 30. The medicinal liquid injection valve 20 includes an inlet portion 23 configured to allow liquid to be introduced from the outside therethrough and an inlet port opening/closing part 24 configured to be detachably coupled to the inlet portion 23. Arrow E1 in FIG. 1 indicates the direction of coupling/decoupling the inlet port opening/closing part 24 with respect to the inlet portion 23.

The extension tube 30 is configured to guide the flow of the priming liquid. The extension tube 30 may guide the movement of the medicinal liquid from the pumping module 10 to the medicinal liquid supply control apparatus 80.

The extension tube 30 is configured to allow the medicinal liquid flowing out from the pumping module 10 according to the pressing in the pumping module 10 to flow therethrough. The upstream end of the extension tube 30 is connected to the pumping module 10. The extension tube 30 includes an upstream connection portion 35 connected to the discharge port portion 11a of the pumping module 10. The extension tube 30 includes the downstream connection portion 36 connected to the end cap 70 or the patient connection unit 60 or 60'. The liquid passing through the upstream sections 31 and 32 of the extension tube 30 flows into the medicinal liquid supply control apparatus 80 through an inlet port of the medicinal liquid supply control apparatus 80, and flows into a downstream section 33 of the extension tube 30 through the outlet port of the medicinal liquid supply control apparatus 80.

The extension tube 30 includes a first connection portion 31 connecting the upstream connection portion 35 and the first extension portion 21 of the medicinal liquid injection valve 20. The extension tube 30 includes a second connection portion 32 interconnecting the second extension portion 22 of the medicinal liquid injection valve 20 and the inlet connection portion 217 of the medicinal liquid supply control apparatus 80. The extension tube 30 includes a third connection portion 33 interconnecting the outlet connection portion 218 of the medicinal liquid supply control apparatus 80 and the downstream connection portion 36.

The medicinal liquid injection apparatus 1 may include at least one connection tube opening/closing module 40. The connection tube opening/closing module 40 may press the outside of the extension tube 30 to block the flow of the liquid at a point in the extension tube 30. The at least one connection tube opening/closing module 40 may include a first opening/closing module 41 capable of changing whether to open or close a point B1 of the first connection portion 31, and a second opening/closing module 42 capable of changing whether to open or close a point B2 of the second connection portion 32. For example, the connection tube opening/closing module 40 may be configured in a clamp shape.

In another embodiment (not illustrated), the medicinal liquid injection apparatus 1 may include a filter module (not illustrated) outside the medicinal liquid supply control apparatus 80 disposed on the extension tube 30, but in the present embodiment, the medicinal liquid supply control apparatus 80 includes a filter to be described later. The filter may include a particle filter for filtering out impurities and/or an air filter for filtering out air bubbles.

A priming step and a medical liquid injection step according to an embodiment will be described as follows. In the priming step according to the embodiment, a saline solution, which is not a medicinal liquid, is used as the priming liquid. In the priming step according to the embodiment, the inlet port opening/closing part 24 is separated from the inlet portion 23, the first connection portion 31 is blocked by the first connection tube opening/closing module 41 (see B1), and the remaining portion of the extension tube 30 except for the first connection portion 31 is opened. Referring to arrows F1, F3, and F4, the priming liquid such as a saline solution flows sequentially through the inlet portion 23, the second extension portion 22, the second connection portion 32, the medicinal liquid supply control apparatus 80, and the third connection portion 33, so that the interior of the extension tube 30 and the interior of the medicinal liquid supply control apparatus 80 are filled with the priming liquid.

After the priming step according to the embodiment, the medicinal liquid injection step according to the embodiment proceeds. In the medicinal liquid injection step according to the embodiment, the inlet port opening/closing part 24 is separated from the inlet portion 23, and the second connection portion 32 is blocked with the second connection tube opening/closing module 42 (see B2), and the first connection tube opening/closing module 41 is separated from the first connection portion 31 to open the first connection portion 31. Referring to arrow F0, as the medicinal liquid flows into the chamber 11 through the medicinal liquid injection valve 20 and the first connection portion 31, the pressing unit 12 moves in the direction Ap2. Thereafter, the inlet port opening/closing part 24 is coupled to the inlet portion 23, and the second connection tube opening/closing module 42 is separated from the second connection portion 32 to open the extension tube 30. Referring to arrows F2, F3, and F4, thereafter, by moving the pressing unit 12 in the pressing direction Ap1, the medicinal liquid may pass sequentially through the upstream sections 31 and 32 of the extension tube, the medicinal liquid supply control apparatus 80, and the downstream section 33 of the extension tube.

A priming step and a medical liquid injection step according to another embodiment will be described as follows. In the priming step according to the other embodiment, a medicinal liquid is used as the priming liquid. In the priming step according to the other embodiment, the chamber 11 is filled with the medicinal liquid, the end cap 70 is connected with the downstream connection portion 36, the inlet portion 23 is blocked with the inlet port opening/closing part 24, and the connection tube opening/closing module 40 is separated from the extension tube 30 to make the extension tube 30 open. Referring to arrows F2, F3, and F4, thereafter, by moving the pressing unit 12 in the pressing direction Ap1, the medicinal liquid, which is the priming liquid, flows sequentially through the upstream sections 31 and 32 of the extension tube, the medicinal liquid supply control apparatus 80, and the downstream section 33 of the extension tube, so that the interior of the extension tube 30 and the interior of the medicinal liquid supply control apparatus 80 are filled with the priming liquid.

After the priming step according to the other embodiment, a medicinal liquid injection step according to the other embodiment is performed. In the medicinal liquid injection step according to the other embodiment, referring to arrows F2, F3, and F4, the pressing unit 12 is further moved in the pressing direction Ap1, so that the medicinal liquid can pass sequentially through the upstream sections 31 and 32 of the extension tube, the medicinal liquid supply control apparatus 80, and the downstream section 33 of the extension tube.

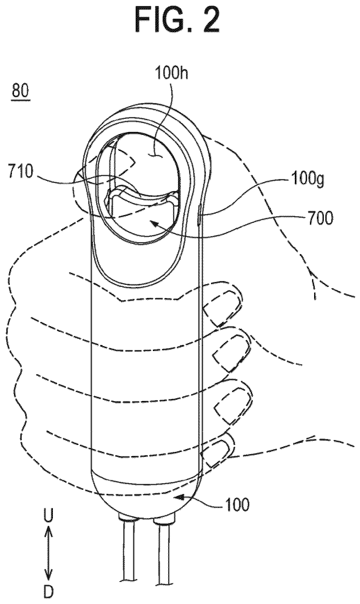

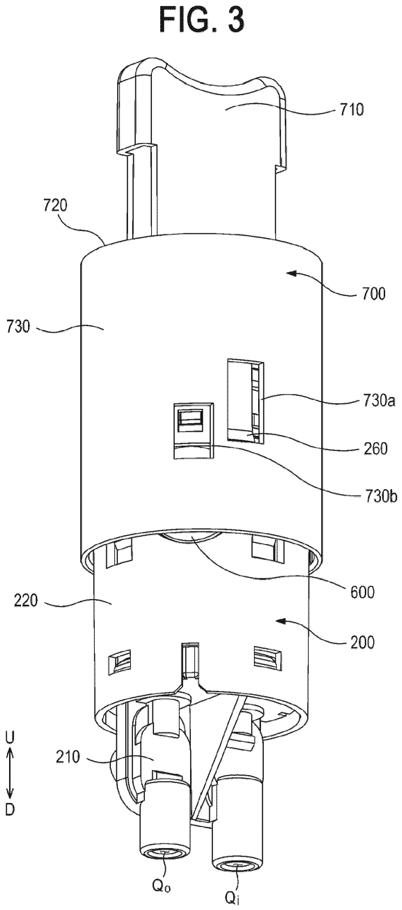

FIG. 2 is a perspective view of a medicinal liquid supply control apparatus 80 according to a first embodiment of the present disclosure, illustrating a figure in which a user operates a button member 700 in a state in which a cotter 1100 has been removed. FIG. 3 is a perspective view illustrating a state in which a case 100 is removed from the medicinal liquid supply control apparatus 80 of FIG. 2.

Referring to FIGS. 1 to 3, the medicinal liquid injection apparatus 1 includes the medicinal liquid supply control apparatus 80 including a medicinal liquid channel connected to the extension tube 30. The medicinal liquid supply control apparatus 80 includes a medicinal liquid channel that guides the flow of a medicinal liquid. The medicinal liquid supply control apparatus 80 may include the inlet connection portion 217 connected to a portion 32 of the extension tube of an upstream side and the outlet connection portion 218 connected to a portion 33 of the extension tube of a downstream side. The medicinal liquid may flow into the medicinal liquid supply control apparatus 80 through an inlet Qi of the inlet connection portion 217 and flow out to the outside of the medicinal liquid supply control apparatus 80 through an outlet Qo of the outlet connection portion 218.

The medicinal liquid supply control apparatus 80 is an apparatus for controlling a dose of a medicinal liquid to be supplied to a patient. The medicinal liquid channel of the medicinal liquid supply control apparatus 80 may be configured in various ways, but in the present embodiment, the medicinal liquid channel includes a first channel P1 to be described later and a second channel P2 to be described later. The first channel P1 guides the medicinal liquid to flow from the inlet Qi to the outlet Qo. In the state in which the channel is not closed by the connection tube opening/closing module 40, a first channel valve 316 of a second embodiment to be described later, or the like, the first channel P1 guides the medicinal liquid to continuously flow from the inlet Qi to the outlet Qo. The second channel P2 guides the medicinal liquid to split at a branching point Qd, which will be described later, of the first channel P1. The second channel P2 guides the split flows of the medicinal liquid to be merged at a merging point Qu, which will be described later, located downstream of the branching point Qd of the first channel P1. The second channel P2 may be configured to be capable of opening/closing.

The medicinal liquid supply control apparatus 80 may include the case 100 defining the external appearance, and a button member 700 provided to be operable by a user. An operation part 710, which is a part of the button member 700, may be exposed to the outside of the case 100. A user (e.g., a patient) may push the operation part 710 to adjust a supply dose of the medicinal liquid. The medicinal liquid supply control apparatus 80 may include a cotter 1100 inserted into the case 100 to prevent the button member 700 from being operated before user's operation. The cotter 1100 is inserted into the case 100 in the state of pushing the operation part 710 downward. When the cotter 1100 is separated from the case 100, the button member 700 moves upward by an elastic member 1000, which will be described later, to be in the state in which the user is capable of pushing the button member 700 downward.

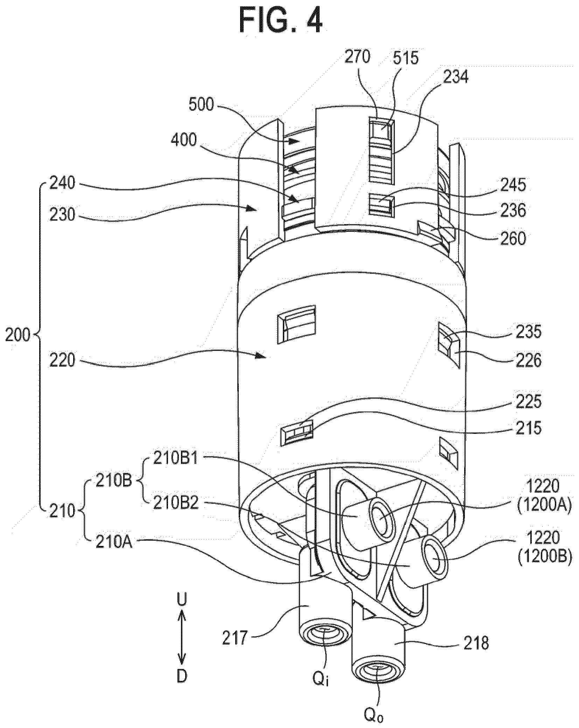

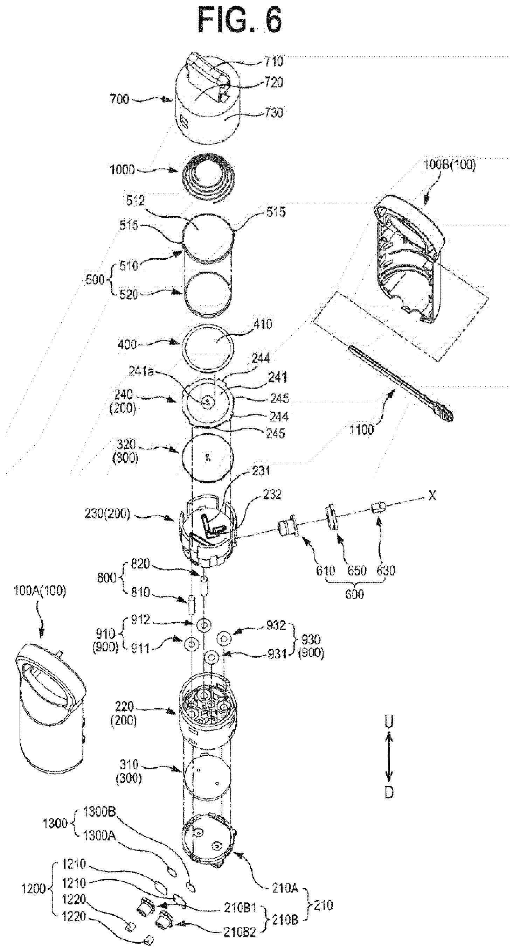

FIG. 4 is a perspective view illustrating a state in which the button member 700 is removed from the medicinal liquid supply control apparatus 80 of FIG. 3. FIG. 5 is an exploded perspective view of the medicinal liquid supply control apparatus 80 of FIG. 1. FIG. 6 is an exploded perspective view of the medicinal liquid supply control apparatus 80 of FIG. 5 viewed from another angle.

Referring to FIGS. 3 to 6, the medicinal liquid supply control apparatus 80 may include a body 200 that defines at least a portion of the medicinal liquid channel. The body 200 may include a plurality of parts 210, 220, 230, and 240. The medicinal liquid supply control apparatus 80 may include at least one sealing plate 300 disposed between adjacent two of the plurality of parts 210, 220, 230, and 240. The medicinal liquid supply control apparatus 80 may include a reservoir 400 that stores a medicinal liquid. The medicinal liquid supply control apparatus 80 may include a reservoir pushing member 500 configured to be capable of pushing the reservoir 400. The medicinal liquid supply control apparatus 80 may include a lock assembly 600 configured to open/close a portion of the medicinal liquid channel.

The medicinal liquid supply control apparatus 80 may include a button member 700 which a user operates. The medicinal liquid supply control apparatus 80 may include a medicinal liquid transfer tube 800 that limits the flow rate of the medicinal liquid per hour. The medicinal liquid supply control apparatus 80 may include at least one sealer 900 disposed between adjacent two of the plurality of parts 210, 220, 230, and 240. The medicinal liquid supply control apparatus 80 may include an elastic member 1000 configured to be elastically deformed when the button member 700 is operated. The medicinal liquid supply control apparatus 80 may include at least one air passage filter 1200 that filters out air in the medicinal liquid channel. The medicinal liquid supply control apparatus 80 may include a hydrophilic boundary filter 1300 disposed on the medicinal liquid channel.

Referring to FIGS. 3 and 4, the body 200 may include a guide protrusion 260 protruding in a direction perpendicular to the upper-lower direction. The button member 700 may be disposed to be slidable up and down along the outer surface of the body 200. The button member 700 may include a sliding portion 730 facing the outer surface of the body 200.

A guide hole 730a into which the guide protrusion 260 is inserted is formed at the sliding portion 730. The guide protrusion 260 may move relatively in the upper-lower direction along the guide hole 730a. The movement direction of the button member 700 with respect to the body 200 is limited in the upper-lower direction by the guide hole 730a and the guide protrusion 260. The sliding portion 730 includes a head insertion portion 730b which defines a groove or hole into which a head 633, which will be described later, of the lock assembly 600 is insertable. The head 633 may be inserted into the groove or hole of the head insertion portion 730b in the released state which will be described later.

The body 200 may include a pushing guide portion 234 for guiding the reservoir pushing member 500 in the moving direction. The pushing guide portion 234 may include a hole or a groove into which a protrusion 515 of the reservoir pushing member 500 is inserted. The hole or groove of the pushing guide portion 234 may extend in the upper-lower direction to guide the protrusion 515 to move in the upper-lower direction along the groove or hole. In the present embodiment, the pushing guide portion 234 is formed at the third part 230, and the protrusion 515 is formed at the upper plate 510.

The body 200 may include a limit portion 270 configured to be capable of being engaged with the reservoir pushing member 500 to limit the upward maximum movement range of the reservoir pushing member 500. The limit portion 270 may include a lower surface that is capable of contacting the protrusion 515 of the reservoir pushing member 500. The limit portion 270 may be an upper end of the pushing guide portion 234. In the present embodiment, the limit portion 270 may be formed at the third part 230.

Referring to FIGS. 4 to 6, the case 100 may include a first case part 100A and a second case part 100B that are coupled to each other. The case 100 may accommodate the body 200 therein. The case 100 may accommodate the parts other than the operation part 710 of the button member 700 therein. A hole 100h into which a user's finger is insertable may be formed at the case 100. The operation part 710 may be exposed in the hole 100h. The cotter 1100 may be inserted into the case 100 to cross the hole 100h in a direction perpendicular to the penetration direction of the hole 100h. A hole 100g into which the cotter 1100 is inserted is formed at the case 100.

The body 200 may include a plurality of parts 210, 220, 230, and 240. The plurality of parts 210, 220, 230, and 240 may be assembled with each other. The plurality of parts 210, 220, 230, and 240 may be sequentially arranged and assembled with each other. The plurality of parts 210, 220, 230, and 240 may be arranged in the upper-lower direction and assembled with each other.

The plurality of parts 210, 220, 230, and 240 may include a first part 210 and a second part 220 that are coupled to each other. The plurality of parts 210, 220, 230, and 240 may include a third part 230 and a fourth part 240 that are coupled to each other. The plurality of parts 210, 220, 230, and 240 may include a second part 220 and a third part 230 that are coupled to each other. According to an embodiment, the first part 210, the second part 220, the third part 230, and/or the fourth part 240 may be referred to as "a filter support part 210", "a branching part 220", "a connection part 230", and/or "a reservoir support part 240" respectively.

The plurality of parts 210, 220, 230, 240 may be hook-coupled to each other. One of the two adjacent parts among the plurality of parts 210, 220, 230, and 240 may include a hook, and the other one may include a hook coupling portion to which the hook is engaged. The hook coupling portion may define a groove or a hole into which the hook is inserted. A plurality of hooks spaced apart from each other may be formed along the circumference of one part, and a plurality of hook coupling portions corresponding to the plurality of hooks may be formed along the circumference of the other part coupled to the one part.

In an embodiment, a first hook 215 of the first part 210 may be coupled to a first hook coupling portion 225 of the second part 220. The third hook 235 of the third part 230 may be coupled to the third hook coupling portion 226 of the second part 220. The fourth hook 245 of the fourth part 240 may be coupled to the fourth hook coupling portion 236 of the third part 230.

In the present embodiment, the at least one sealing plate 300 includes a lower sealing plate 310 disposed between the first and second parts 210 and 220 located adjacent to each other, and an upper sealing plate 320 disposed between the third and fourth parts 230 and 240 located adjacent to each other, but in another embodiment (not illustrated), only one sealing plate may be provided and a sealing plate may be disposed between two parts different from the present embodiment. The sealing plate 310 may be referred to as "a lower sealing plate 310" and the sealing plate 320 may be referred to as an "upper sealing plate 320."

One of two parts (the first and second parts 210 and 220 or the third and fourth parts 230 and 240), which are located adjacent to each other with the sealing plate 300 interposed therebetween, is recessed in a direction opposite the direction in contact with the surface of the sealing plate 300 to form at least one channel groove 222 and/or 232 extending along the surface of the sealing plate 300. In the present embodiment, of the two adjacent parts 210 and 220, the channel groove 222 is formed at the second part 220, but in another embodiment (not illustrated), the channel groove is formed at the first part 210. In the present embodiment, of the two adjacent parts 230 and 240, the third part 230 is provided with the channel groove 232, but in another embodiment (not illustrated), the channel groove may be formed at the fourth part 240. Hereinafter, a description will be made with reference to an embodiment in which the channel groove 222 is formed at the second part 220 and the channel groove 232 is formed at the third part 230, but the disclosure is not limited thereto.

The channel grooves 222 and 232 are covered with the sealing plates 300 and constitute a portion of the medicinal liquid channel. An inlet Qi is located at an upstream starting point of the medicinal liquid channel, and an outlet Qo is located at a downstream end point of the medicinal liquid channel.

A channel groove 222 may be formed at one 220 of the two adjacent parts 210 and 220, and an inlet Qi and an outlet Qo may be formed at the other one 210. The channel groove 232 may be formed at one of the two adjacent parts 230 and 240, and the reservoir may be supported by the other one 240.

One of two parts (the first and second parts 210 and 220 or the third and fourth parts 230 and 240) located adjacent to each other with the sealing plate 300 interposed therebetween may include channel guide rib 221 and/or 231 protruding toward the surface of the sealing plate 300 and extending along the surface. The channel guide ribs 221 and 231 partition the channel grooves 222 and 223. The channel guide ribs 221 and 231 are in contact with the sealing plate. As the channel guide ribs 221 and 231 press the sealing plate 300, the channels formed by the channel grooves 222 and 223 may be more stably sealed.

One or more extension holes 220h and 230h that are connected to the grooves 222 and 223 and constitute a portion of the medicinal liquid channel may be formed at one of two parts (the first and second parts 210 and 220 or the third and fourth parts 230 and 240) located adjacent to each other with the sealing plate 300 interposed therebetween. The at least one extension hole 220h or 230h may extend in the upper-lower direction. The at least one extension hole 220h may include a plurality of extension holes 220h1, 220h2, 220h3, and 220h4. The at least one extension hole 230h may include a plurality of extension holes 230h1, 230h2, 230h3, and 230h4.

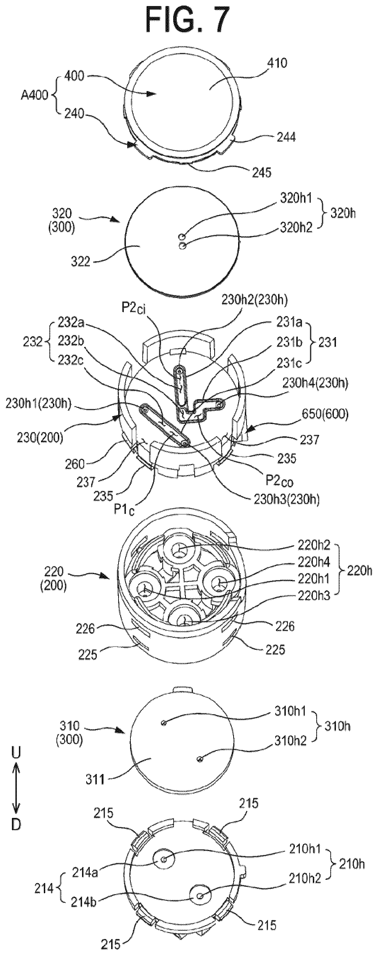

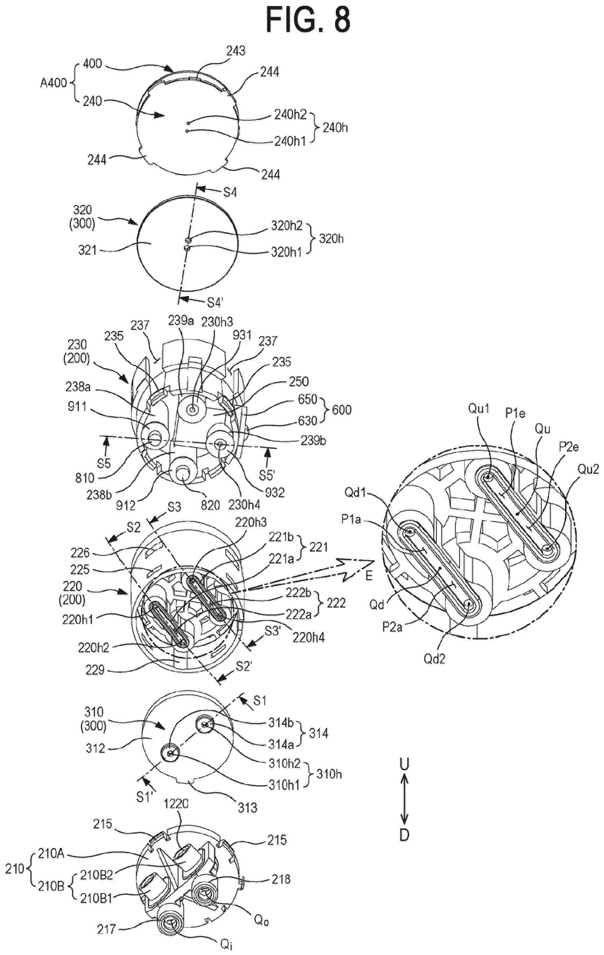

FIG. 7 is an exploded perspective view illustrating only some of the components of FIG. 5, in which unlike FIG. 5, some components are illustrated in the state of being assembled with each other. FIG. 8 is an exploded perspective view of the state of FIG. 7 viewed from another angle, in which an enlarged view (E) of a portion is illustrated.

Points Qd, Qd1, Qd2, Qu, Qu1, and Qu2 located in the medicinal liquid channel will be described with reference to the enlarged view (E) of FIG. 8. The branching point Qd is a point at which the medicinal liquid flowing along the first channel P1 is split into the second channel P2. The merging point Qu is a point at which the medicinal liquid flowing along the second channel P2 merges into the first channel P1. On the first channel PI, a first split-flow guide point Qd1 is located between the branching point Qd and the merging point Qu. On the second channel P2, a first split-flow guide point Qd1 is located between the branching point Qd and the merging point Qu. On the first channel PI, a first merging guide point Qu1 is located between the branching point Qd and the merging point Qu. On the first channel PI, the first merging guide point Qu1 may be located between the first split-flow guide point Qd1 and the merging point Qu. On the second channel P2, a second merging guide point Qu2 is located between the branching point Qd and the merging point Qu. On the second channel P2, the second merging guide point Qu2 may be located between the second split-flow guide point Qd2 and the merging point Qu. The branching point Qd, the first split-flow guide point Qd1, and the second split-flow guide point Qd2 may be located in a channel groove 222a. The merging point Qu, the first merging guide point Qu1, and the second merging guide point Qu2 may be located in the channel groove 222b.

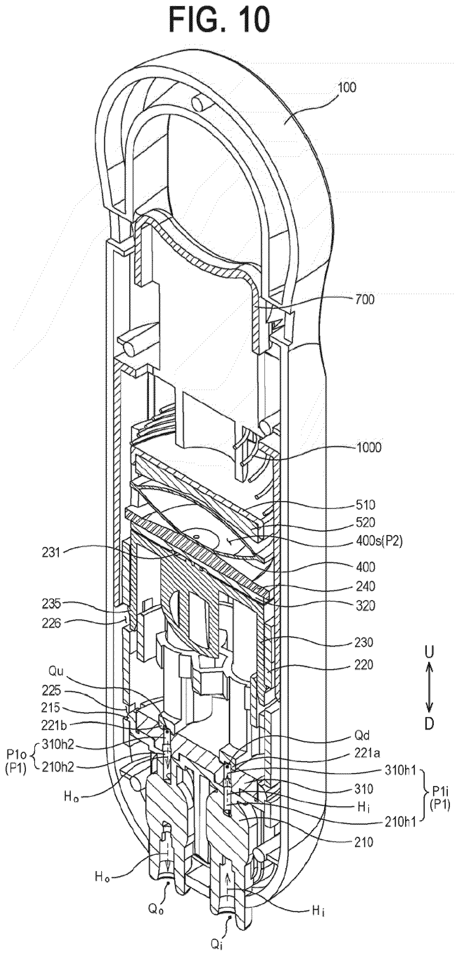

Referring to FIGS. 5 to 8, the first part 210 may define a portion of an inlet channel P1i and a portion of an outlet channel P1o, which will be described later, of the first channel P1 (see FIG. 10). An inlet Qi and an outlet Qo may be formed at the first part 210. The first part 210 may include the inlet connection portion 217 defining the inlet Qi and the outlet connection portion 218 defining the outlet Qo. The first part 210 may include a filter body 210A and a filter cap 210B, which are coupled to each other.

In an embodiment, the boundary filter 1300 may be disposed in the filter body 210A. The filter cap 210B may be disposed in a direction in which one surface of the boundary filter 1300 faces.

In an embodiment, one or more air passage filters 1200 may be disposed on the first part 210. One or more air passage filters 1200 may be fixed to the filter cap 210B. The first part 210 may define an air passage branched from the medicinal liquid channel. The first part 210 may define at least one vent hole (not illustrated) located at a point where the air passage is connected to the external space. The vent hole 910h may be formed at the filter cap 210B.

The filter cap 210B may include an inlet filter cap 210B1 that defines a vent hole through which air split from the inlet channel P1i is discharged. The filter cap 210B may include an outlet filter cap 210B2 that forms a vent hole through which air split from the outlet channel P1o is discharged.

At least one channel hole 210h constituting a portion of the medicinal liquid channel may be formed at the first part 210. The at least one channel hole 210h may include an inlet channel hole 210h1 constituting a portion of the inlet channel P1i. The at least one channel hole 210h may include an outlet channel hole 210h2 constituting a portion of the outlet channel P1o. One end of the inlet channel hole 210h1 defines an inlet Qi, and the other end of the inlet channel hole 210h1 faces the sealing plate 310. One end of the outlet channel hole 210h2 defines an outlet Qo, and the other end of the outlet channel hole 210h2 faces the sealing plate 310.

An insertion groove 214 into which a protrusion 314 of the sealing plate 310 is inserted is formed at the first part 210. The insertion groove 214 is formed by being recessed in a direction opposite to the direction facing the sealing plate 310. The insertion groove 214 may include an upstream insertion groove 214a in which the other end of the inlet channel hole 210h1 is located. The insertion groove 214 may include a downstream insertion groove 214b in which the other end of the outlet channel hole 210h2 is located.

The sealing plate 310 may be in contact with the lower surface of the second part 220. The first part 210 may be coupled to the lower side of the second part 220. The medicinal liquid transfer tube 800 may be disposed on the upper portion of the second part 220. The second part 220 may support the lower end of the medicinal liquid transfer tube 800. The second part 220 may support one end of a first medicinal liquid transfer tube 810. The second part 220 may support one end of a second medicinal liquid transfer tube 820.

At least one channel groove 222 may be formed at the second part 220. The at least one channel groove 222 may include at least one of a branching channel groove 222a and a merging channel groove 222b. The branching channel groove 222a may be covered with the sealing plate 310 and configure branching channels P1a and P2a. The merging channel groove 222b may be covered with the sealing plate 310 and configure merging channels P1e and P2e.

The branching point Qd, the first split-flow guide point Qd1, and the second split-flow guide point Qd2 may be located in the branching channel groove 222a. The branching channel groove 222a may extend in a direction perpendicular to the penetrating direction (upper-lower direction) of a connection hole 310a of the sealing plate 310. The upper end of the connection hole 310a faces the branching point Qd.

The merging point Qu, the first merging guide point Qu1, and the second merging guide point Qu2 may be located in the merging channel groove 222b. The merging channel groove 222a may extend in a direction perpendicular to the penetrating direction (upper-lower direction) of a connection hole 310b of the sealing plate 310. The upper end of the connection hole 310b faces the merging point Qu.

The branching channel groove 222a and the merging channel groove 222b may be disposed to be horizontally spaced apart from each other. The branching channel groove 222a and the merging channel groove 222b may be disposed in parallel to each other.

The second part 220 may include the channel guide rib 221 protruding downward to come into contact with the sealing plate 310. The channel guide rib 221 may include a branching channel guide 221a that partitions the branching channel groove 222a. The channel guide rib 221 may include a merging channel guide 221b that partitions the merging channel groove 222b.

At least one extension hole 220h constituting a portion of the medicinal liquid channel is formed at the second part 220. The extension hole 220h may extend in the upper-lower direction. The lower end of the extension hole 220h is located in the channel groove 222.

The at least one extension hole 220h may include an extension hole 220h1 constituting a portion of a first upstream extension channel P1b to be described later, and an extension hole 220h2 constituting a portion of a second upstream extension channel P2b to be described later. The lower end of the extension hole 220h1 may face the first split-flow guide point Qd1. The lower end of the extension hole 220h2 may face the second split-flow guide point Qd2.

The at least one extension hole 220h may include an extension hole 220h3 constituting a portion of a first downstream extension channel P1d to be described later, and an extension hole 220h4 constituting a portion of a second downstream extension channel P2d to be described later. The lower end of the extension hole 220h3 may face the first merging guide point Qu1. The lower end of the extension hole 220h4 may face the second merging guide point Qu2.

The second part 220 may be formed in a cylindrical shape extending up and down as a whole. The sealing plate 310 may be disposed on the lower side of the second part 220. A guide groove 229 into which the guide protrusion 313 of the sealing plate 310 is inserted may be formed at the second part.

The sealing plate 320 may be in contact with the upper surface of the third part 230. The second part 220 may be coupled to the lower side of the third part 230. The fourth part 240 may be coupled to the upper side of the third part 230. The medicinal liquid transfer tube 800 may be disposed on the lower portion of the third part 230. The third part 230 may support the upper end of the medicinal liquid transfer tube 800. The third part 230 may support the other end of the first medicinal liquid transfer tube 810. The third part 230 may support the other end of the second medicinal liquid transfer tube 820.

At least one channel groove 232 may be fromed at the third part 230. The at least one channel groove 232 may include at least one of an inlet channel groove 232a, an outlet channel groove 232b, and a connection channel groove 232c. The inlet channel groove 232a may be covered with the sealing plate 320 and constitute a portion of the reservoir inlet channel P2ci. The outlet channel groove 232b may be covered with the sealing plate 320 and constitute a portion of the reservoir outlet channel P2co. The connection channel groove 232c may be covered with the sealing plate 320 and constitute a first connection guide channel P1c.

The inlet channel groove 232a may extend in a direction perpendicular to the penetrating direction (upper-lower direction) of a connection hole 320h1 of the sealing plate 320. The lower end of the connection hole 320h1 faces the inlet channel groove 232a.

The outlet channel groove 232b may extend in a direction perpendicular to the penetrating direction (upper-lower direction) of a connection hole 320h2 of the sealing plate 320. The lower end of the connection hole 320h2 faces the outlet channel groove 232b.

The lower surface of the sealing plate 320 may cover the connection channel groove 232c. The sealing plate 320 may cover the entire connection channel groove 232c so that the connection channel groove 232c does not have an upwardly connected channel.

The inlet channel groove 232a and the outlet channel groove 232b may be disposed to be horizontally spaced apart from each other. The inlet channel groove 232a, the outlet channel groove 232b, and the connection channel groove 232c may be disposed to be horizontally spaced apart from each other.

The third part 230 may include the channel guide rib 231 protruding upward to come into contact with the sealing plate 320. The channel guide rib 231 may include an inlet channel guide 231a that partitions the inlet channel groove 232a. The channel guide rib 231 may include an outlet channel guide 231b that partitions the outlet channel groove 232b. The channel guide rib 231 may include a connection channel guide 231c that partitions the connection channel groove 232c.

At least one extension hole 230h constituting a portion of the medicinal liquid channel may be formed at the third part 230. The extension hole 230h may extend in the upper-lower direction. The upper end of the extension hole 230h is located in the channel groove 232.

The at least one extension hole 230h may include an extension hole 230h1 constituting a portion of a first upstream extension channel P1b to be described later, and an extension hole 230h2 constituting a portion of a second upstream extension channel P2b to be described later. The upper end of the extension hole 230h1 and the upper end of the extension hole 230h2 may be located in the connection channel groove 232c.

The at least one extension hole 230h may include an extension hole 230h3 constituting a portion of a first downstream extension channel P1d to be described later, and an extension hole 230h4 constituting a portion of a second downstream extension channel P2d to be described later. The upper end of the extension hole 230h3 may be located in the inlet channel groove 232a. The upper end of the extension hole 230h4 may be located in the outlet channel groove 232b.

The extension hole 220h1 of the second part 220, a capillary channel 810p of the first medicinal liquid transfer tube 810, and the extension hole 230h1 of the third part 230 may be sequentially connected to each other to define the first upstream extension channel P1b. The extension hole 220h2 of the second part 220, a capillary channel 820p of the second medicinal liquid transfer tube 820, and the extension hole 230h2 of the third part 230 may be sequentially connected to each other to define the second upstream extension channel P2b. The extension hole 230h3 of the third part 230 and the extension hole 220h3 of the second part 220 may be sequentially connected to each other to define the first downstream extension channel P1d. The extension hole 230h4 of the third part 230 and the extension hole 220h4 of the second part 220 may be sequentially connected to each other to define the first downstream extension channel P1d.

A guide groove 237 that guides a coupling direction for assembling the fourth part 240 is formed at the third part 230. The guide groove 237 may be formed by being recessed in a downward direction in a portion that defines the peripheral surface of the third part 230. The coupling guide 244 protruding laterally from the fourth part 240 may be engaged with the guide groove 237.

At least one of the second part 220 and the third part 230 may include transfer tube seating portions 238a and 238b on which the medicinal liquid transfer tube 800 is seated. The transfer tube seating portions 238a and 238b may include a first transfer tube seating portion 238a on which the first medicinal liquid transfer tube 810 is seated, and a second transfer tube seating portion 238b on which the second medicinal liquid transfer tube 820 is seated. The transfer tube seating portions 238a and 238b may define a hole into which the medicinal liquid transfer tube 800 is inserted, and the hole into which the medicinal liquid transfer tube 800 is inserted may communicate with the above-described extension hole. In the present embodiment, the transfer tube seating portions 238a and 238b are formed at the third part 230, wherein the extension hole 230h1 communicates with the hole of the first transfer tube seating portion 238a, and the extension hole 230h2 communicates with the second transfer tube seating portion 238b.

At least one of the second part 220 and the third part 230 may include the channel forming portions 239a and 239b extending the above-described extension holes in the upper-lower direction. The channel forming portions 239a and 239b may extend in parallel to the transfer tube seating portion 238a. In the present embodiment, the channel forming portions 239a and 239b are formed at the third part 230, wherein the first channel forming portions 239a extends the extension hole 230h1 in the upper-lower direction, and the second channel forming portion 239b extends the extension hole 230h4 in the upper-lower direction.

The third part 230 may be formed in a cylindrical shape extending up and down as a whole. The lower portion of the third part 230 may be inserted into and coupled to the upper portion of the second part 220. The sealer 900 may be sandwiched between the second part 220 and the third part 230. The second part 220 and the third part 230 may be coupled to each other such that the medicinal liquid transfer tube 800 is disposed therein.

The reservoir support part (the fourth part) 240 may define a portion of a second connection guide channel P2c to be described later. A connection hole 240h may be formed at the reservoir support part 240. The connection hole 240h may penetrate the reservoir support part 240 in the upper-lower direction. The connection hole 240h may be formed at the central portion of the reservoir support part 240.

The connection hole 240h may include an inlet connection hole 240h1 and an outlet connection hole 240h2. The inlet connection hole 240h1 and the outlet connection hole 240h2 constitute a portion of the second channel P2.

The reservoir support part 240 may be formed in a plate shape. The reservoir support part 240 may be formed in a circular shape as a whole when viewed from above. The reservoir support part 240 may be inserted into and coupled to the upper portion of the third part 230. The reservoir support part 240 may include a coupling guide 244 protruding outward from an edge of the reservoir support part 240 to be perpendicular to the upper-lower direction. A plurality of coupling guides 244 spaced apart from each other may be provided along the edge of the reservoir support part 240.

A sealing plate 300 is sandwiched between two adjacent parts of the plurality of parts 210, 220, 230, and 240 to be in contact with the two adjacent parts. The medicinal liquid channel penetrates the two adjacent parts and the sealing plate 300 sandwiched between the two adjacent parts. A connection hole 310h or 320h that is connected to a channel groove 222 or 232 and constitutes a portion of the medicinal liquid channel is formed at the sealing plate 300. The connection hole 310h or 320h may penetrate the sealing plate 300 in the upper-lower direction.

Any one 220 or 230 of two parts (the first and second parts 210 and 220 or the third and fourth parts 230 and 240) located adjacent to each other with the sealing plate 300 interposed therebetween may form a channel groove 222 or 232, and a portion of the medicinal liquid channel may be formed at the other one 210 or 240. A portion of the medicinal liquid channel of the other part 210 or 240 of the two parts located adjacent to each other with the sealing plate 300 interposed therebetween, the connection hole 310h or 320h, and the channel groove 222 or 232 may be sequentially connected to each other.

The sealing plate 300 may be formed in a plate shape. The sealing plate 300 may be formed in a circular shape when viewed from above. The sealing plate 300 may be made of a material that is more easily elastically deformable than the material of two parts located adjacent to each other with the sealing plate 300 interposed therebetween. In the present embodiment, the material of the two parts adjacent to each other includes a synthetic resin, and the material of the sealing plate 300 includes rubber or silicone. The sealing plate 300 is elastically deformed by being pressed by the two adjacent parts, thereby stably preventing the medicinal liquid inside the channel grooves 222 and 223 from leaking. In the present embodiment, the sealing plate 300 may be elastically deformed to be depressed by being pressed by the channel guide ribs 221 and 231.

The at least one sealing plate 300 may include a lower sealing plate 310 sandwiched between the first part 210 and the second part 220 to be in contact with the first part 210 and the second part 220. The lower sealing plate 310 may include a cover surface 311 that is in contact with one 220 of the two adjacent parts 210 and 220 that has a channel groove 222 and an auxiliary cover surface 312 that is in contact with the other one 220. The cover surface 311 may form the upper surface, and the auxiliary cover surface 312 may form the lower surface.

An upstream connection hole 310h1 connected to the branching channel groove 222a and a downstream connection hole 310h2 connected to the merging channel groove 222b may be formed at the lower sealing plate 310. The inlet channel hole 210h1 of the first part 210 and the upstream connection hole 310h1 may be sequentially connected to each other to define the inlet channel P1i (see FIG. 10). The downstream connection hole 310h2 and the outlet channel hole 210h2 of the first part 210 may be sequentially connected to each other to define the outlet channel P1o (see FIG. 10).

The lower sealing plate 310 may include at least one protrusion 314 protruding from the auxiliary cover surface 312 to a direction facing the first part 210. The lower end of the connection hole 310h may be located at the lower end of the protrusion 314. The at least one protrusion 314 may include an upstream protrusion 314a corresponding to the upstream connection hole 310h1 and a downstream protrusion 314b corresponding to the downstream connection hole 310h2.

The lower sealing plate 310 may include a guide protrusion 313 protruding outward from an edge to be perpendicular to the upper-lower direction. The guide protrusion 313 may be inserted into the guide groove 229 of the body 200.

At least one sealing plate 300 may include an upper sealing plate 320 that is sandwiched between the third part (the connection part) 230 and the fourth part (the reservoir support part) 240 to be in contact with the second part 220 and the fourth part 240. The upper sealing plate 320 may include a cover surface 321 that is in contact with one 230 of the two adjacent parts 230 and 240 that has a channel groove 232 and an auxiliary cover surface 322 that is in contact with the other one 220. The cover surface 321 may form the lower surface, and the auxiliary cover surface 312 may form the upper surface.

a first connection hole 320h1 that constitutes a portion of the second channel P2 and is connected to the inlet connection hole 240h1 of the reservoir support part 240 may be formed at the upper sealing plate 320. a second connection hole 320h2 that constitutes a portion of the second channel P2 and is connected to the outlet connection hole 240h2 of the reservoir support part 240 may be formed at the upper sealing plate 320. The lower end of the first connection hole 320h1 may face the inlet channel groove 232a of the third part 230, and the lower end of the second connection hole 320h2 may face the outlet channel groove 232b of the third part 230.

The reservoir pushing member 500 is configured to be capable of pressing the reservoir 400. The reservoir pushing member 500 may be disposed above the reservoir 400. The reservoir pushing member 500 may be configured to move downward to press the reservoir 400. When the reservoir pushing member 500 moves downward, the reservoir 400 is pushed between the reservoir pushing member 500 and the reservoir support part 240, so that the medicinal liquid in the internal space 400s of the reservoir 400 may be discharged to the outside of the reservoir 400. The reservoir pushing member 500 may include an upper plate 510 and a reservoir pressing portion 520.

The upper plate 510 may be coupled to the upper side of the reservoir pressing portion 520. The upper plate 510 may be formed in a circular shape as a whole when viewed from above. The upper plate 510 may include a coupling surface 511 coupled to the upper surface of the reservoir pressing portion 520, and a pushing surface 512 pressed by the button member 700. The upper plate 510 may include a side guide 513 that extends along the circumference of the coupling surface 511 to be in contact with the edge of the reservoir pressing portion 520. The upper plate 510 may include a protrusion 515 protruding outward from an edge to be perpendicular to the upper-lower direction.

The reservoir pressing portion 520 includes a lower surface 521 that presses the reservoir 400. The reservoir pressing portion 520 may be formed of a material that is more flexible than that of the upper plate 510. In the present embodiment, the material of the upper plate 510 may include a synthetic resin, and the material of the reservoir pressing portion 520 may include rubber or silicone.

The lower surface 521 of the reservoir pressing portion 520 may include an upwardly concave surface. The concave surface of the lower surface 521 may be formed to correspond to the convex surface of the upper surface 241 of the reservoir support part 240. According to an embodiment, the concave surface may have a curvature different from that of the convex surface, and may include a medicinal liquid guide portion 521a that forms an upwardly recessed groove in the concave surface. A detailed description thereof will be given later.

The button member 700 may be configured to be upwardly spaced apart from the reservoir pushing member 500. The button member 700 may be configured to be capable of pressing the reservoir pushing member 500 downward. When the user pushes the operation part 710 of the button member 700 downward, the button member 700 may move downward and come into contact with the upper surface of the reservoir pushing member 500. When the button member 700 further moves downward in the state of being in contact with the upper surface of the reservoir pushing member 500, the button member 700 and the reservoir pushing member 500 may integrally move downward, and the reservoir pushing member 500 may press the reservoir 400 downward.

The button member 700 may be coupled to the body 200 to be movable in the upper-lower direction along the outer surface of the body 200. The button member 700 may include a sliding portion 730 surrounding the peripheral surface of the body 200. The sliding portion 730 may be formed in a cylindrical shape as a whole. The button member 700 may include an upper surface portion 720 extending horizontally from an upper portion of the sliding portion 730. The button member 700 may include an operation part 710 protruding upward from the upper surface portion 720.

The elastic member 1000 may be disposed between the reservoir pushing member 500 and the button member 700. The elastic member 1000 may be configured to be elastically deformed when the reservoir pushing member 500 and the button member 700 come close to each other. When the user releases the button member 700 after pushing the button member 700 downward, the button member 700 may move upwardly to be spaced apart from the reservoir pushing member 500 by the restoring force of the elastic member 1000. At this time, the reservoir pushing member 500 and the reservoir support part 240 may maintain the state of being located close to each other.

The elastic member 1000 may use one of various known methods of exerting an elastic force. For example, the elastic member 1000 may include various types of members such as a compression spring, a tension spring, a torque spring, and an air spring, or may include a member made of a material such as rubber to be elastically compressed.

The medicinal liquid supply control apparatus 80 may include the at least one medicinal liquid transfer tube 800 having capillary channels 810p and 820p constituting a portion of the medicinal liquid channel. The medicinal liquid transfer tube 800 is coupled to the body 200. The medicinal liquid transfer tube 800 may have a function of limiting the flow rate of the medicinal liquid per hour. As an example, the medicinal liquid transfer tube 800 may include a capillary tube. As another example, the medicinal liquid transfer tube 800 may include a polymer microtube. In addition, the medicinal liquid transfer tube 800 may be configured in various shapes having a capillary channel using various materials. For example, the capillary channel 820p may have a diameter of about 0.04 to 0.08 mm, thereby limiting the flow rate of a medicinal liquid per hour.

The at least one medicinal liquid transfer tube 800 may include the first medicinal liquid transfer tube 810 that defines the capillary channel 810p constituting a portion of the first channel P1. The first medicinal liquid transfer tube 810 may be disposed in at least one of the plurality of parts 210, 220, 230, and 240. In the present embodiment, the capillary channel 810p constitutes a portion of the first upstream extension channel P1b, but in another embodiment (not illustrated), the capillary channel 810p may constitute a portion of the first downstream extension channel P1d.

The at least one medicinal liquid transfer tube 800 may include the second medicinal liquid transfer tube 820 that defines the capillary channel 820p constituting a portion of the second channel P2. The second medicinal liquid transfer tube 820 may be disposed in at least one of the plurality of parts 210, 220, 230, and 240. In the present embodiment, the capillary channel 820p constitutes a portion of the second upstream extension channel P2b, but in another embodiment (not illustrated), the capillary channel 820p may constitute a portion of the second downstream extension channel P2d.

At least a portion of the medicinal liquid transfer tube 800 may be in contact with the inner surface of the body 200. The medicinal liquid transfer tube 800 may be disposed to pass through a transfer tube sealer 910.

The sealer 900 may be formed in a ring shape. The sealer 900 may be formed of an elastic material such as rubber. In the present embodiment, the sealer 900 is disposed to be sandwiched between the second part 220 and the second part 220.

The at least one sealer 900 may include at least one transfer tube sealer 910 disposed between the outer surface of the medicinal liquid transfer tube 800 and the inner surface of the housing 81. The transfer tube sealer 910 may block the medicinal liquid from flowing between the outer surface of the medicinal liquid transfer tube 800 and the inner surface of the body 200. The transfer tube sealer 910 may surround the circumference of the medicinal liquid transfer tube 800. The at least one transfer tube sealer 910 may include a first transfer tube sealer 911 disposed between the outer surface of the first medicinal liquid transfer tube 810 and the inner surface of the body 200. The at least one transfer tube sealer 910 may include a second transfer tube sealer 912 disposed between the outer surface of the second medicinal liquid transfer tube 820 and the inner surface of the body 200.

The at least one sealer 900 may include at least one connection sealer 930 that is sandwiched between two parts 220 and 230 that are coupled to each other to prevent the leakage of the medicinal liquid between the two parts 220 and 230. The connection sealer 930 is disposed in a portion of the medicinal liquid channel in which the medicinal liquid transfer tube 800 is not disposed. The at least one connection sealer 930 may include a first connection sealer 931 disposed in the first downstream extension channel P1d defined by the two parts 220 and 230 that are coupled to each other. The at least one connection sealer 930 may include a second connection sealer 932 disposed in the second downstream extension channel P2d defined by the two parts 220 and 230 that are coupled to each other.

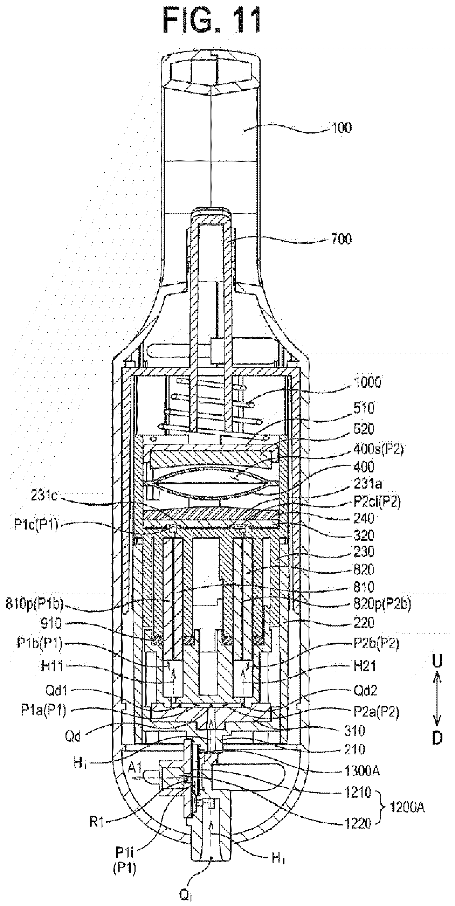

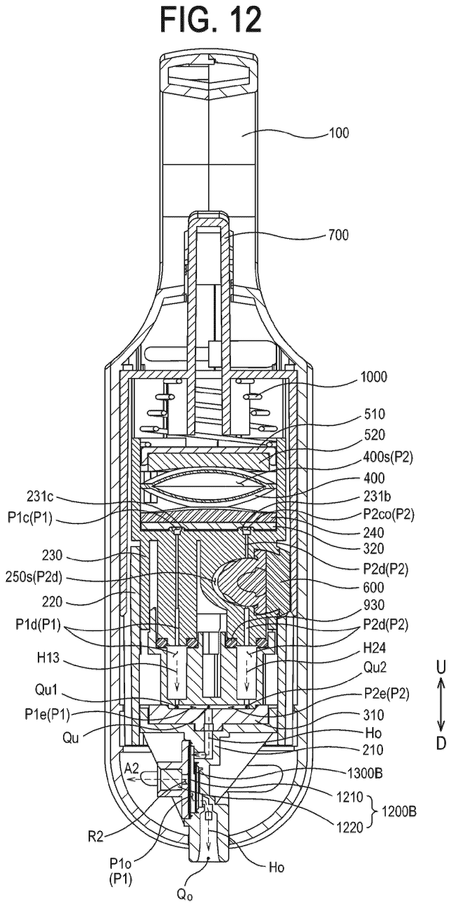

The medicinal liquid supply control apparatus 80 may include at least one hydrophobic air passage filter 1200. The air passage filter 1200 blocks the passage of the medicinal liquid but allows air to pass therethrough. The air passage filter 1200 may be disposed on the filter support part 210. The filter support part 210 may define a passage R1 of air split from the inlet channel P1i (see FIG. 11). The filter support part 210 may define a passage R2 of air split from the outlet passage P1o (see FIG. 12). Arrow A1 in FIG. 11 illustrates a direction in which air passes along the passage R1, and arrow A2 in FIG. 12 illustrates a direction in which air passes along the passage R2.

The at least one air passage filter 1200 may include an upstream air passage filter 1200A located upstream of the branching point Qd on the first channel P1. The upstream air passage filter 1200A may be configured to discharge air in the first channel P1 to the outside. The upstream air passage filter 1200A may include a first air passage filter 1210 disposed at a boundary between the air passage R1 and the inlet channel P1i. The upstream air passage filter 1200A may further include a second air passage filter 1220 disposed on the air passage R1.

The at least one air passage filter 1200 may include a downstream air passage filter 1200B located downstream of the merging point Qu on the first channel P1. The downstream air passage filter 1200B may be configured to discharge air in the first channel P1 to the outside. The downstream air passage filter 1200B may include a first air passage filter 1210 disposed at a boundary between the air passage R2 and the outlet channel P1o. The upstream air passage filter 1200A may further include a second air passage filter 1220 disposed on the air passage R2.

The air passage filter 1200 may include a first air passage filter 1210 and a second air passage filter 1220 that are sequentially disposed on the air passages R1 and R2. The second air passage filter 1220 is configured to allow air that has passed through the first air passage filter 1210 to pass therethrough. The second air passage filter 1220 may perform a function of preventing the internal medicinal liquid from flowing out even when a pore, a bonded portion, or the like of the first air passage filter 1210 is damaged.

The second air passage filter 1220 may be produced by processing the same material as the first air passage filter 1210 or a porous plastic material. As an example, the second air passage filter 1220 may be configured such that a hydrophobic porous plastic resin material fills cross-sectional areas of some of the air passages R1 and R2. For example, the material of the second air passage filter 1220 is available from Porex Corporation (web site: https://www.porex.com) of Fairburn, GA 30213, USA. A product released under the name of Porex Hydrophobic Vents from Porex Corporation is available, wherein the product is made of a material of polyethyl polytetrafluoroethylene.

The medicinal liquid supply control apparatus 80 may include at least one hydrophilic boundary filter 1300 disposed on the medicinal liquid channel. The boundary filter 1300 may be disposed to cross the medicinal liquid channel. The at least one boundary filter 1300 may include an upstream boundary filter 1300A disposed between the upstream air passage filter 1200A and the branching point Qd on the inlet channel P1i. The at least one boundary filter 1300 may include a downstream boundary filter 1300B disposed between the outlet Qo and the downstream air passage filter 1200B on the outlet channel P1o.

The boundary filter 1300 is configured to act as a pressure interface between the upstream channel portion and the downstream channel portion with reference to the boundary filter 1300 when the boundary filter is wetted with the medicinal liquid. By the boundary filter 1300, the upstream channel portion and the downstream channel portion may have different internal pressures. For example, the boundary filter 1300 may be configured in at least one of a mesh structure and a fiber structure. The boundary filter 1300 may additionally have a function of filtering impurities.

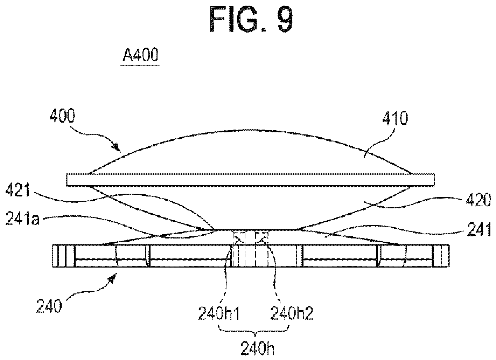

FIG. 9 is an elevation view of the reservoir assembly A400 of FIG. 7. Referring to FIG. 9, the reservoir assembly A400 is a component including the assembly of the reservoir 400 and the reservoir support part 240. The medicinal liquid supply control apparatus 80 may include the reservoir assembly A400.

The reservoir (reservoir) 400 defines an internal space 400s for storing a medicinal liquid. In the medicinal liquid supply control apparatus 80, the internal space 400s of the reservoir 400 is located on the medicinal liquid channel. In the medicinal liquid supply control apparatus 80, the internal space 400s of the reservoir 400 may be located on a second channel P2.

The reservoir 400 is configured such that the volume of the internal space 400s can be changed. As the volume of the internal space 400s of the reservoir 400 increases, the medicinal liquid may be stored, and as the volume of the internal space 400s of the reservoir 400 decreases, the stored medicinal liquid may be discharged.

The reservoir 400 may include an upper first film 410 and a lower second film 420 coupled to each other. For example, each of the first film 410 and the second film 420 may be a polyvinyl chloride (PVC) film. The first film 410 and the second film 420 may be coupled at the top and bottom of respective edges thereof to define the internal space 400s between the first film 410 and the second film 420.

At least one hole 420h connected to the internal space 400s may be formed at one portion 421 of the outer surface of the reservoir 400. The at least one hole 420h may include an inlet hole 420h1 and an outlet hole 420h2. The inlet hole 420h1 may constitute a portion of the reservoir inlet channel P2ci, and the outlet hole 420h2 may constitute a portion of the reservoir outlet channel P2co.

The one portion 421 of the reservoir 400 may be fixed to the reservoir support part 240. The one portion 421 may be located at the central portion of the reservoir 400. The one portion 421 may be located at the center of the lower surface of the reservoir 400. The one portion 421 may be located on the second film 420.

The reservoir support part 240 may be disposed below the reservoir 400. The reservoir 400 may be fixed to the upper surface 241 of the reservoir support part 240. The upper surface 241 of the reservoir support part 240 may include an upwardly convex surface.

The one portion 421 of the reservoir 400 may be fixed to one portion 241a of the upper surface of the reservoir support part 240. The one portion 241a may be located at the central portion of the reservoir support part 240. The one portion 421 of the reservoir 400 may be located at the center of the lower surface of the reservoir 400, and the convex surface of the reservoir support part 240 may protrude from a position corresponding to the center of the reservoir 400.

The convex surface of the reservoir support part 240 may have an upwardly convex curvature. The reservoir support part 240 may be configured such that the convex surface has an uppermost end in the central portion. The convex surface of the reservoir support part 240 may have the uppermost end in the one portion 241a of the reservoir support part 240.

An inlet connection hole 240h1 that constitutes a portion of the medicinal liquid channel and is connected to the inlet hole 420h1 of the reservoir 400 may be formed at the reservoir support part 240. An outlet connection hole 240h2 that constitutes a portion of the medicinal liquid channel and is connected to the outlet hole 420h2 may be formed at the reservoir support part 240.

The inlet connection hole 240h1 may constitute a portion of the reservoir inlet channel P2ci. The inlet connection hole 240h1 may be connected to the inlet hole 420h1 of the reservoir 400. The inlet hole 420h1 and the inlet connection hole 240h1 may be vertically connected to each other. The inlet connection hole 240h1 may be connected to the first connection hole 320h1 of the sealing plate 320. The inlet connection hole 240h1 and the first connection hole 320h1 may be vertically connected to each other.

The outlet connection hole 240h2 may constitute a portion of the reservoir outlet channel P2co. The outlet connection hole 240h2 may be connected to the outlet hole 420h2 of the reservoir 400. The outlet hole 420h2 and the outlet connection hole 240h2 may be vertically connected to each other. The outlet connection hole 240h2 may be connected to the second connection hole 320h2 of the sealing plate 320. The outlet connection hole 240h2 and the second connection hole 320h2 may be vertically connected to each other.

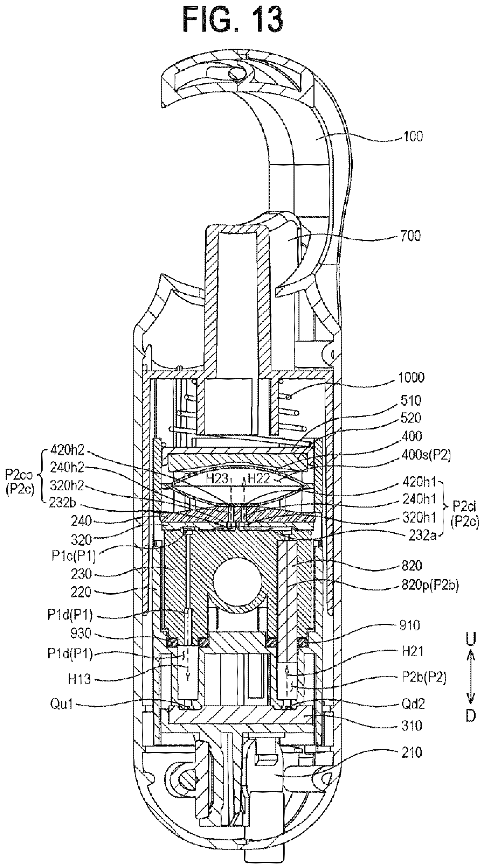

FIG. 10 is a perspective view illustrating a cross section of the medicinal liquid supply control apparatus 80 taken along line S1-S1' in FIG. 8. FIG. 11 is a cross-sectional view of the medicinal liquid supply control apparatus 80 taken along line S2-S2' in FIG. 8. FIG. 12 is a cross-sectional view of the medicinal liquid supply control apparatus 80 taken along line S3-S3' in FIG. 8. FIG. 13 is a cross-sectional view of the medicinal liquid supply control apparatus 80 taken along line S4-S4' in FIG. 8.

Referring to FIGS. 10 to 13, the medicinal liquid channel may include a first channel P1 that guides the medicinal liquid to flow from the inlet Qi to the outlet Qo. The medicinal liquid channel may include a second channel P2 that guides the medicinal liquid to split at the branching point of the first channel P1 and guides the split medicinal liquid to merge at the merging point Qu located downstream of the branching point Qd of the first channel P1. The second channel P2 may be configured to be capable of opening/closing.

The inlet channel P1i, the first split-flow guide channel P1a, the first upstream extension channel P1b, the first connection guide channel P1c, the first downstream extension channel P1d, the first merging guide channel P1e, and the outlet passage P1o may be sequentially connected to each other to form the first channel P1. The first capillary channel 810p may form a portion of the first upstream extension channel P1b.

The second split-flow guide channel P2a, the second upstream extension channel P2b, the reservoir inlet channel P2ci, the internal space 400s of the reservoir 400, the reservoir outlet channel P2co, the second downstream extension channel P2d, and the second merging guide channel P2e may be sequentially connected to each other to form the second channel P2. The second capillary channel 820p may constitute a portion of the second upstream extension channel P2b. An insertion space 250s to be described later may constitute a portion of the second downstream extension channel P2d.

Referring to FIGS. 10 and 11, the inlet channel P1i is a channel section from the inlet Qi to the branching point Qd. After flowing along the inlet channel P1i (see arrow Hi), the medicinal liquid is split at the branching point Qd and flows along split-flow channels P1a and P2a. The first split-flow guide channel P1a is a channel section from the branching point Qd to the first split-flow guide point Qd1. After flowing along the first split-flow guide channel P1a, the medicinal liquid flows along the first upstream extension channel P1b (see arrow H11). The second split-flow guide channel P2a is a channel section from the branching point Qd to the second split-flow guide point Qd2. After flowing along the second split-flow guide channel P2a, the medicinal liquid flows along the second upstream extension channel P2b (see arrow H21).

Referring to FIGS. 11 and 12, on the first channel PI, a first connection guide channel P1c is located between the branching point Qd and the merging point Qu. The first connection guide channel P1c may interconnect the downstream end of the first upstream extension channel P1b and the upstream end of the first downstream extension channel P1d. After sequentially flowing along the first upstream extension channel P1b and the first connection guide channel P1c, the medicinal liquid flows along the first downstream extension channel P1d (arrow H13).

Referring to FIGS. 12 and 13, on the second channel P2, a second connection guide channel P2c is located between the branching point Qd and the merging point Qu. The second connection guide channel P2c may interconnect the downstream end of the second upstream extension channel P2b and the downstream end of the second downstream extension channel P2d. After sequentially flowing along the second upstream extension channel P2b and the second connection guide channel P2c, the medicinal liquid flows along the second downstream extension channel P2d (arrow H24).

Referring to FIG. 13, the second connection guide channel P2c may include a reservoir inlet channel P2ci located between the branching point Qd and the internal space 400s of the reservoir 400 on the second channel P2. The inlet channel groove 232a of the third part 230, the first connection hole 320h1 of the sealing plate 320, the inlet connection hole 240h1 of the fourth part 240, and the inlet hole 420h1 of the reservoir 400 may be sequentially connected to each other to form the reservoir inlet channel P2ci.

Referring to FIG. 13, the second connection guide channel P2c may include a reservoir outlet channel P2co located between the merging point Qu and the internal space 400s of the reservoir 400 on the second channel P2. The outlet hole 420h2 of the reservoir 400, the outlet connection hole 240h2 of the fourth part 240, the second connection hole 320h2 of the sealing plate 320, and the outlet channel groove 420h2 of the third part 230 may be sequentially connected to each other to form the reservoir outlet channel P2co. The medicinal liquid in the internal space 400s of the reservoir 400 flows along the reservoir outlet channel P2co *(see* arrow H23), and then flows along the second downstream extension channel P2d (*see* arrow H24).

Referring to FIGS. 10 and 12, the split medicinal liquid split by the split-flow channels P1a and P2a are merged through the merging channels P1e and P2e to flow along the outflow channel P1o (*see* arrow Ho). The first merging guide channel P1e is a channel section from the first merging guide point Qu1 to the merging point Qu. After flowing along the first downstream extension channel P1d (*see* arrow H13), the medicinal liquid flows along the first merging guide channel P1e. In addition, the second merging guide channel P2e is a channel section from the second merging guide point Qu2 to the merging point Qu. After flowing along the second downstream extension channel P2d (*see* arrow H24), the medicinal liquid flows along the second merging guide channel P2e. The outlet channel P1o is a channel section from the merging point Qu to the outlet Qo. After flowing along the merging channels P1e or P2e, the medicinal liquid flows along the outlet channel P1o (*see* arrow Ho).

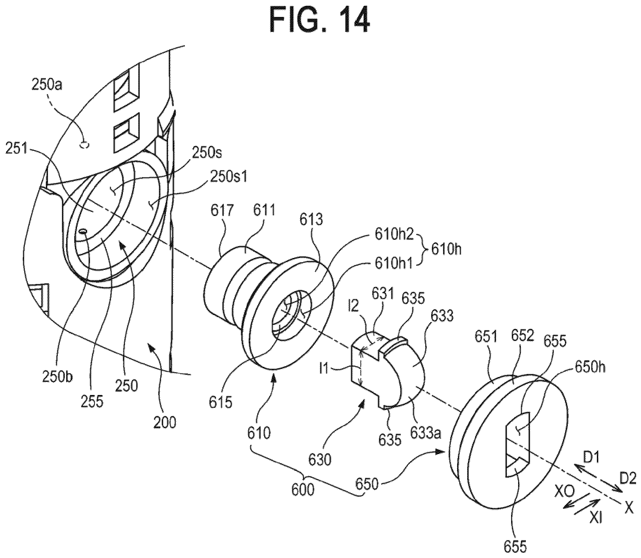

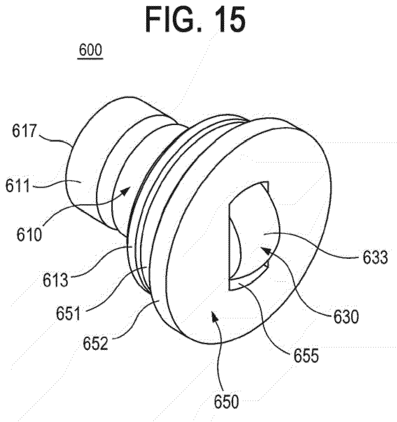

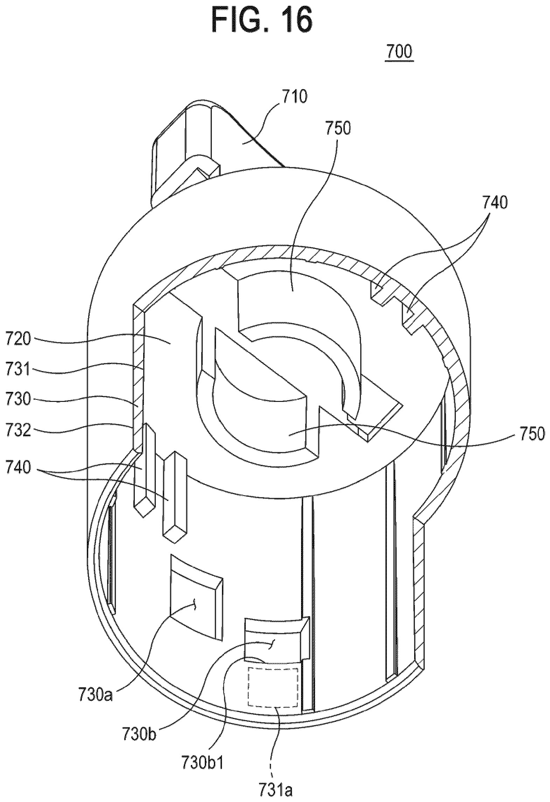

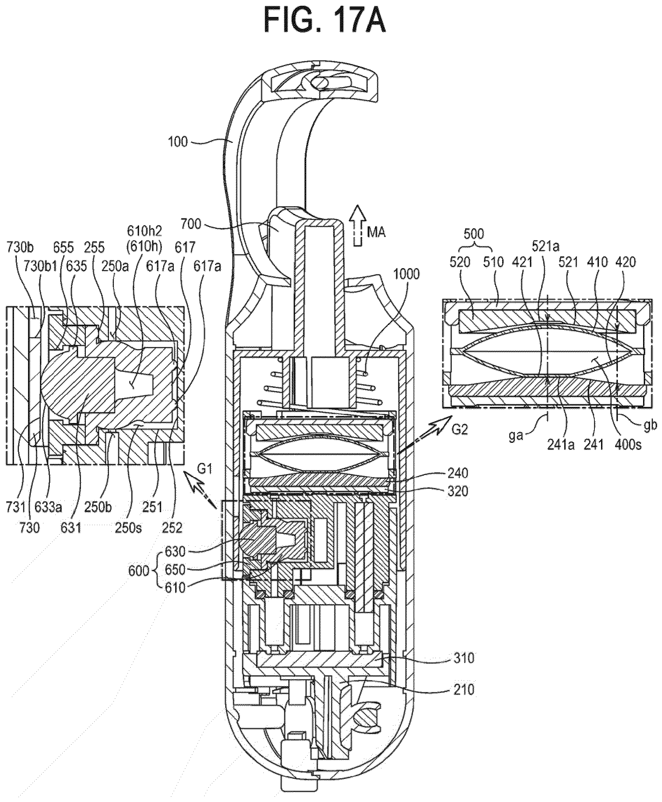

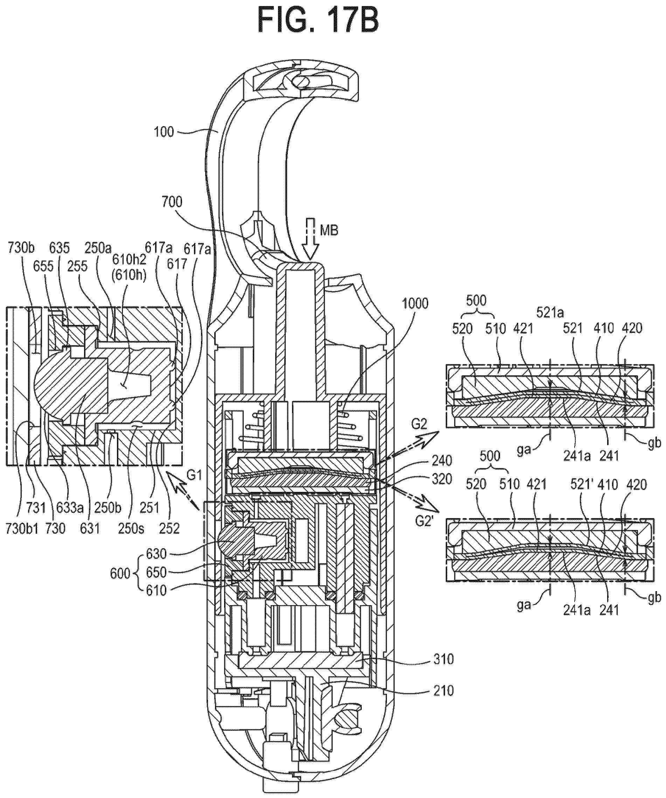

FIG. 14 is an exploded perspective view of the lock assembly 600 and the body 200 of FIG. 5. FIG. 15 is a perspective view illustrating a state in which the lock assembly 600 of FIG. 14 is coupled. FIG. 16 is a perspective view of the button member 700 of FIG. 5 in a state in which a portion thereof is cut away. FIGS. 17A and 17B are cross-sectional views of the medicinal liquid supply control apparatus 80 taken along line S5-S5' in FIG. 8, FIG. 17A is a view illustrating a locked state, and FIG. 17B is a view illustrating a released state.

In describing the lock assembly 600, a central axis X, which is a virtual axis, may be defined. In the present embodiment, the central axis X extends in a direction perpendicular to the upper-lower direction, but the present disclosure is not limited thereto. In the present disclosure, one of the extension directions of the central axis X may be defined as "a first direction D1" and a direction opposite to the first direction D1 may be defined as "a second direction D2." The "radially outward direction XO" used in the present disclosure means a direction away from the central axis X, and the "radially inward direction XI" means a direction approaching the central axis X.

Referring to FIGS. 14 to 17B, the body 200 may form an insertion space 250s that is recessed in the first direction D1. The insertion space 250s may have an opening 250s1 to the second direction D2.

The body 200 may include a space forming part 250 that forms the insertion space 250s. In the present embodiment, the space forming part 250 is provided in the third part 230. The space forming part 250 may include surfaces 251 and 252 defining the insertion space 250s. The surfaces 251 and 252 may include a peripheral surface 251 defining the periphery of the insertion space 250s about the central axis X. The surfaces 251 and 252 may include an end surface 252 defining the end of the insertion space 250s in the first direction D1. The end surface 252 may extend from the end of the peripheral surface 251 in the first direction D1.

The space forming part 250 may include an engagement step 255 facing the second direction D2. A stopper 613 of the lock valve 610 may be in contact with the engagement step 255. The engagement step 255 may extend from the end of the peripheral surface 251 in the second direction D2.

A first hole 250a and a second hole 250b may be located in the surfaces 251 and 252 that define the insertion space 250s of the body 200. The first hole 250a and the first hole 250a may be located on the peripheral surface 251 of the surfaces 251 and 252. The first hole 250a and the second hole 250b may be formed to face each other with the lock valve 610 interposed therebetween.

The first hole 250a, the insertion space 250s, and the second hole 250b may be sequentially connected to constitute a portion of the medicinal liquid channel. The first hole 250a, the insertion space 250s, and the second hole 250b may be sequentially connected to constitute a portion of the second channel P2. In the present embodiment, the first hole 250a, the insertion space 250s, and the second hole 250b are sequentially connected to form the second downstream extension channel P2d. The medicinal liquid may flow through a gap between the outer surface of the lock valve 610 and the surfaces 251 and 252 of the space forming part 250.

The lock assembly 600 includes a lock valve 610 inserted into the insertion space 250s. The lock valve 610 may block the opening 250s1 of the insertion space 250s. The lock valve 610 may include a valve groove 610h recessed in the first direction D1. The valve groove 610h may be recessed in the first direction from the surface of the lock valve 610 in the second direction D2. The valve groove 610h may be recessed along the central axis X.

The lock assembly 600 includes a lock pin 630 inserted into the valve groove 610h. The lock pin 630 may be configured to elastically deform the lock valve 610. The state in which the lock pin 630 moves in the first direction D1 may be defined as "a locked state" and the state in which the lock pin 630 moves in the second direction D2 may be defined as "a released state." In the locked state, the lock valve 610 blocks at least one of the first hole 250a and the second hole 250b (*see* FIG. 17A). In the present embodiment, in the locked state, the lock valve 610 blocks both the first hole 250a and the second hole 250b. In the released state, the lock valve 610 opens the first hole 250a and the second hole 250b (*see* FIG. 17B).

The lock valve 610 may be formed of a material that is more flexible than the material of the lock pin 630. The lock valve 610 may be formed of a material that is more flexible than the material of the space forming part 250. In the present embodiment, the material of the lock pin 630 and the material of the space forming part 250 may include a synthetic resin, and the material of the lock valve 610 may include rubber or silicone. The lock valve 610 may be configured to be elastically deformed or elastically restored in response to the movement of the lock pin 630 in the first direction D1 or the second direction D2.

The lock valve 610 may include an insertion portion 611 that is inserted into the insertion space 250s and forms a valve groove 610h. The valve groove 610h may be formed in the center of the insertion portion 611. The insertion portion 611 may be formed in a cylindrical shape. The peripheral surface facing the radially outward direction XO of the insertion portion 611 may face the peripheral surface 251 of the space forming part 250. A shielding surface 617 facing the first direction D1 of the insertion portion 611 may face the end surface 252 of the space forming part 250.

The lock valve 610 may include an end portion 617a that is in contact with the surfaces 251 and 252 of the body 200 in the first direction D1. The end portion 617a may be in contact with the end surface 252 of the space forming part 250. The end portion 617a may form the end of the lock valve 610 in the first direction D1. The end portion 617a may protrude from the shielding surface 617 in the first direction D1.

The lock valve 610 may include a stopper 613 extending from the insertion portion 611 in the radially outward direction XO. The stopper 613 may extend in the circumferential direction about the central axis X. The stopper 613 may be seated on the body 200. The stopper 613 is seated on the body 200 to block the opening 250s1 of the insertion space 250s. The surface of the stopper 613 facing the first direction D1 may be in contact with the engagement step 255 of the space forming part 250.

The lock valve 610 may include a pin seating portion 615 disposed in the valve groove 610h. The pin seating portion 615 may have a surface in the second direction D2 that is capable of contacting the surface of the lock pin 630 in the first direction D1. In the locked state, the engagement protrusion 635 of the lock pin 630 may come into contact with the surface of the pin seating portion 615 in the second direction D2. When changed from the released state to the locked state, the lock pin 630 moves in the first direction D1 in the state in which the engagement protrusion 635 and the pin seating portion 615 are in contact with each other, and thus the lock valve 610 may be elastically deformed. When changed from the locked state to the released state, the lock valve 610 may be elastically restored and the pin seating portion 615 may push the engagement protrusion 635 in the second direction D2.

The valve groove 610h may include a first recessed portion 610h1 and a second recessed portion 610h2 sequentially arranged in the first direction D1. The first recessed portion 610h1 and the second recessed portion 610h2 may be connected to each other. The first recessed portion 610h1 may be located in the second direction D2 with reference to the pin seating portion 615. The second recessed portion 610h2 may be located in the first direction D1 with reference to the pin seating portion 615. The second recessed portion 610h2 may be narrower than the first recessed portion 610h1 in the radially inward direction XI. The circumferential length of the second recessed portion 610h2 about the central axis X is shorter than the circumferential length of the first recessed portion 610h1 about the central axis X.

The lock pin 630 may include a pushing portion 631 inserted into the valve groove 610h. The pushing portion 631 may be inserted into the second recessed portion 610h2. The pushing portion 631 may protrude from the head 633 in the first direction.

On a cross section perpendicular to the first direction D1, the pushing portion 631 may have a rectangular cross section. In the cross section of the pushing portion 631 perpendicular to the first direction D1, the length 11 in opposite directions in which the first hole 250a and the second hole 250b are disposed may be longer than the length 12 in the direction perpendicular to the opposite directions. Through this, in the locked state, the lock valve 610 is more easily elastically deformed and stretched in the opposite directions in which the first hole 250a and the second hole 250b are disposed, so that the first hole 250a and the second hole 250b can be well-blocked. In the present embodiment, the length 11 is a length in the upper-lower direction.

The lock pin 630 may include a head 633 disposed in the second direction D2 of the pushing portion 631. The head 633 may form the end of the lock pin 630 in the second direction D2. The head 633 may be configured to be capable of contacting the button member 700. In the locked state, the head 633 may come into contact with the contact surface 731a of the button member 700 and may be pushed in the first direction D1. In the released state, the head 633 may be inserted into the head insertion portion 730b of the button member 700.

An inclined surface 633a extending in a direction between the first direction D1 and the downward direction may be formed on the surface of the head 633 in the second direction D2. The button member 700 may move in the upper-lower direction. When the button member 700 moves upward, the head 633 may come into contact with the contact surface 731a of the button member 700.

The lock pin 630 may include an engagement protrusion 635 protruding in the radially outward direction XO. A pair of engagement protrusions 635 protruding in opposite directions may be provided. The engagement protrusions 635 may be disposed between the pushing portion 631 and the head 633. The surfaces of the engagement protrusions 635 in the first direction D1 are contactable with the pin seating portion 615 of the lock valve 610. The surfaces of the engagement protrusion 635 in the second direction D2 are capable of contacting the surface of the engagement portion 655 of the lock cap 650 in the first direction D1.

In the locked state, the engagement protrusions 635 may be in contact with the pin seating portion 615, and in the released state, the engagement protrusions 635 may be spaced apart from the pin seating portion 615 in the second direction D2. In the released state, the engagement protrusions 635 may be in contact with the engagement portion 655, and in the locked state, the engagement protrusions 635 may be spaced apart from the engagement portion 655 in the first direction D1.

The lock assembly 600 may include a lock cap 650 that pushes the stopper 613 of the lock valve 610 and is seated on the body 200. The lock cap 650 may be fixed to the body 200. The material of the lock cap 650 may include a synthetic resin.

The lock cap 650 may include a valve fixing portion 651 that pushes the stopper 613 of the lock valve 610 in the first direction D1. The valve fixing portion 651 may extend in the circumferential direction about the central axis X.

The lock cap 650 may include a seating portion 652 that is seated on the edge of the opening 250s1 of the body 200. The surface of the seating portion 652 in the first direction D1 may be fixed to the body 200. The seating portion 652 may be located in the second direction D2 of the valve fixing portion 651 and extend in the radially outward direction XO. The seating portion 652 may extend in the circumferential direction about the central axis X.

The lock cap 650 may include an engagement portion 655 protruding in the radially inward direction XI. The engagement portion 655 may be engaged with the lock pin 630 to limit the maximum position at which the lock pin 630 is movable in the second direction D2. A pair of engagement portions 655 protruding to face each other may be provided. The engagement portions 655 are disposed on one side of the guide hole 650h.

The lock cap 650 may form a guide hole 650h through which the head 633 of the lock pin 630 passes. The guide hole 650h penetrates the lock cap 650 along the central axis X. The guide hole 650h is formed in a shape corresponding to the shape of the head 633 viewed in the first direction D1, and may guide the movement direction of the lock pin 630.

Referring to FIG. 16, the button member 700 may be disposed to be movable in the upper-lower direction perpendicular to the first direction D1 with respect to the body 200. When the button member 700 completes the upward movement, the button member 700 may be in the locked state, and when the button member 700 completes the downward movement, the button member 700 may be in the released state. When changed from the released state to the locked state, the button member 700 may push the lock pin 630 in the first direction D1. When changed from the locked state to the released state, the button member 700 may release the pushing of the lock pin 630.

The sliding portion 730 of the button member 700 may surround the body 200. The sliding portion 730 may include an inner surface 731 facing the outer surface of the body 200 and an outer surface 732 opposite to the inner surface 731. The inner surface 731 may move upward and downward along the outer surface of the body 200.

The button member 700 may include a contact surface 731a facing the first direction D1. The contact surface 731a may be located on the inner surface 731. The contact surface 731a may come into contact with the head 633 of the lock pin 630. A head insertion portion 730b, which is a hole or a groove recessed in the second direction D2 may be formed at the button member 700. The head insertion portion 730b may be formed at the sliding portion 730. The head insertion portion 730b may be located above the contact surface 731a. In the locked state, the contact surface 731a pushes the lock pin 630 in the first direction D1. In the released state, the head 633 may be inserted into the head insertion portion 730b in the second direction.

When changed from the released state to the locked state, the lower end 730b1 of the head insertion portion 730b may move upward while sliding on the inclined surface 633a of the head 633, and the lock pin 630 may move in the first direction D1. When changed from the locked state to the released state, the lower end 730b1 of the head insertion portion 730b may move downward while sliding on the inclined surface 633a of the head 633, and the head 633 may be inserted into the head insertion portion 730b.

The button member 700 may include a guide rib 740 configured to guide the movement in the upper-lower direction with respect to the body 200. The guide rib 740 may protrude inward from the inner surface 731 and extend in the upper-lower direction.

The button member 700 may include a pushing bar 750 protruding downward from the upper surface portion 720. The pushing bar 750 may be configured to be in contact with the upper surface of the reservoir pushing member 500. The pushing bar 750 may push the reservoir pushing member 500 downward. The pushing bar 750 may be upwardly spaced apart from the reservoir pushing member 500 by the restoring force of the elastic member 1000.

Referring to FIGS. 17A and 17B, the lock valve 610 may be configured to be elastically deformed by the lock pin 630 when changed from the released state to the locked state. When changed from the locked state to the released state, the lock pin 630 may move in the second direction D2 by the elastic restoring force. In the present embodiment, when changed from the locked state to the released state, the lock pin 630 may move in the second direction D2 by the elastic restoring force of the lock valve 610. In another embodiment (not illustrated), a separate elastic member such as a spring is disposed between the lock valve 610 and the lock pin 630 so that, when the lock pin 630 moves in the first direction D1, the elastic member may be elastically deformed, and the lock pin 630 may be pushed in the second direction D2 by the elastic restoring force of the elastic member.

The lock valve 610 may be configured such that the valve groove 610h is elastically deformed to open when the released state is changed to the locked state, and such that when the locked state is changed to the released state, the valve groove 610h is elastically restored to be narrowed. The elastically deformed lock valve 610 may block at least one of the first hole 250a and the second hole 250b. The lock valve 610 may be configured such that, when the released state is changed to the locked state, the insertion portion 611 is elastically deformed while protruding toward a position at which at least one of the first hole 250a and the second hole 250b is located. In the present embodiment, the insertion portion 611 may be elastically deformed and protrude in the upper-lower direction to block the first hole 250a and the second hole 250b.

The lock valve 610 may be configured such that in the released state, the medicinal liquid flows through a space between the outer surface of the insertion portion 611 and the surfaces 251 and 252 of the body 200. The medicinal liquid introduced into the insertion space 250s through the first hole 250a may flow out from the insertion space 250s through the second hole 250b.

The reservoir 400 may be located upstream of the insertion space 250s on the medicinal liquid channel. The reservoir 400 may define an internal space 400s configured to store a medicinal liquid. The medicinal liquid in the internal space 400s may be introduced into the insertion space 250s through the first hole 250a.

The reservoir pushing member 500 may be configured to be capable of pressing the reservoir 400 downward. The button member 700 may be configured to press the reservoir pushing member 500 downward when changed from the locked state to the released state. In the released state, the first hole 250a and the second hole 250b are opened (*see* enlarged views G1 in FIGS. 17A and 17B).

Referring to FIG. 17B, when the user pushes the operation part 710 downward (MB), the button member 700 pushes the reservoir pushing member 500 downward, and the reservoir pushing member 500 pushes the reservoir 400 downward. In addition, the head 633 of the lock pin 630 is inserted into the head insertion portion 730b of the button member 700 so that the lock valve 610 is elastically restored, and the first hole 250a and the second hole 250b are opened. The medicinal liquid inside the pressed reservoir 400 is discharged to the outside of the reservoir 400 and sequentially passes through the first hole 250a, the insertion space 250s, and the second hole 250b, and merges into the first channel P1.

Referring to FIG. 17A, when the user releases the operation part 710, the button member 700 moves upward from the reservoir pushing member 500 by the restoring force of the elastic member 1000 (MA). The button member 700 is engaged with the case 100 and is disposed at the upward maximum movement position. Thereafter, when the reservoir 400 is inflated while the internal space 400s of the reservoir 400 is filled with the medicinal liquid, the reservoir pushing member 500 may be moved upward by the reservoir 400.

One of the upper surface 241 of the reservoir support part 240 and the lower surface 521 of the reservoir pushing member 500 may include a convex surface that is convex toward the other one, and the other one may include a concave surface corresponding to the convex surface. In the present embodiment, the upper surface 241 of the reservoir support part 240 includes the convex surface, and the lower surface 521 of the reservoir pushing member 500 includes the concave surface.

Referring to the enlarged views G2 and G2' of FIGS. 17A and 17B, a gap ga in the upper-lower direction between the upper surface 241 of the reservoir support part 240 and the lower surface 521 of the reservoir pushing member 500 at a position corresponding to the one portion 421 of the reservoir 400 may be larger than a gap gb in the upper-lower direction between the upper surface 241 and the lower surface 521 at a position different from the position corresponding to the one portion 421. The one portion 421 of the reservoir 400 may be located at the center of the lower surface of the reservoir 400, and the gap ga in the upper-lower direction between the convex surface and the concave surface in the central portion of the reservoir 400 may be larger than the gap gb in the upper-lower direction between the convex surface and the concave surface in the edge portion of the reservoir 400. Through this, the medicinal liquid may be discharged through the outlet hole 420h2 located in the one portion 421 of the reservoir 400 while minimizing the remaining amount of the medicinal liquid inside the reservoir 400.

In the state in which the reservoir pushing member 500 is moved upward, the gap ga may be larger than the gap gb. The lower surface 521 of the reservoir pushing member 500 may be made of a material that is more flexible than that of the reservoir support part 240. When the reservoir pushing member 500 moves downward and the edge of the reservoir pushing member 500 is pressed by the edge of the reservoir support part 240, the lower surface 521 of the reservoir pushing member 500 is elastically deformed so that the difference between the gap ga and the gap gb may be reduced or eliminated.

In an embodiment referring to the enlarged view G2 of FIG. 17B, the reservoir pushing member 500 may include a medicinal liquid guide portion 521a that forms a groove recessed upwardly in the center of the lower surface 521. Through this, it is possible to make the gap ga larger than the gap gb.

In another embodiment referring to the enlarged view G2' of FIG. 17B, the one portion 421 of the reservoir 400 is located at the center of the lower surface of the reservoir 400, and on a cross section crossing the center of the reservoir 400 up and down, the concave surface of the lower surface 521' of the reservoir pushing member 500 may have a larger curvature than that of the convex surface. Through this, it is possible to make the gap ga larger than the gap gb.

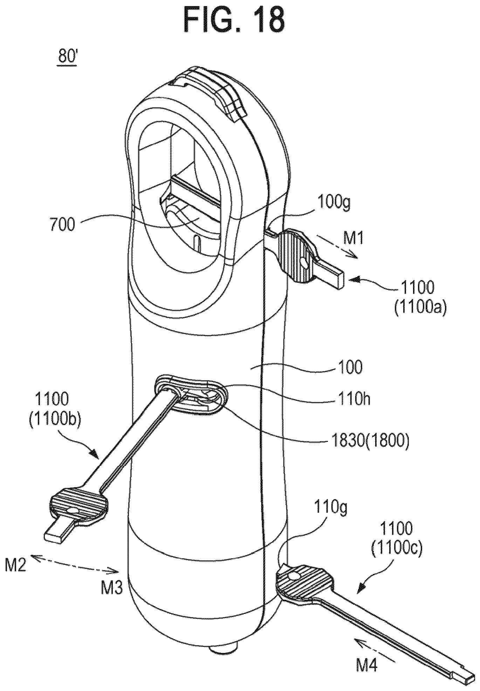

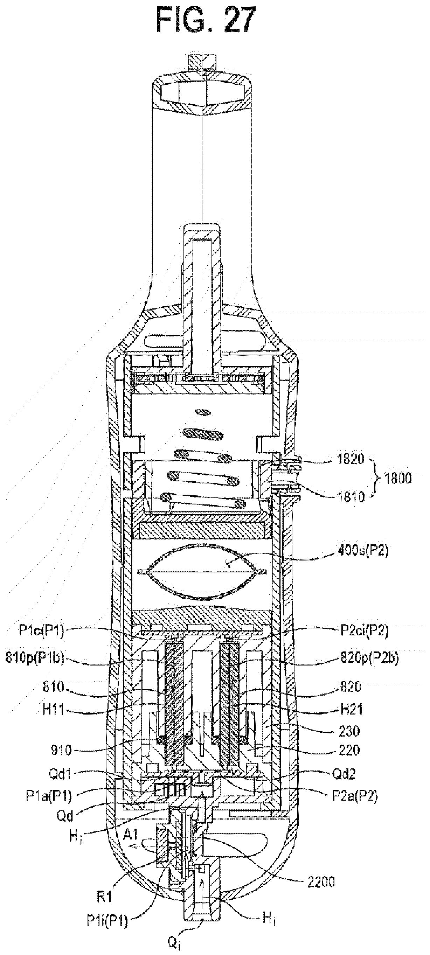

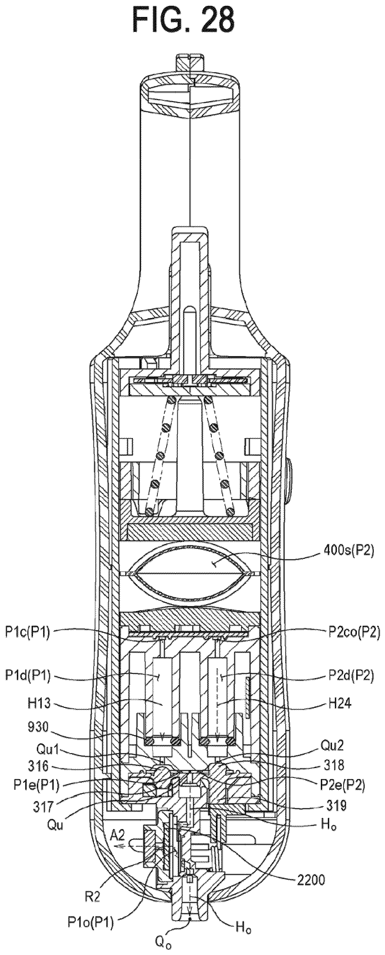

The configuration and mechanism of a medicinal liquid supply control apparatus 80' of a second embodiment corresponding to the configuration and mechanism of the medicinal liquid supply control apparatus 80 of the first embodiment described above with reference to FIGS. 1 to 17B are illustrated in FIGS. 18 to 30 in which the same numbers and symbols are indicated, and a description thereof will be omitted. That is, the description of the above-described first embodiment is applicable to the numbers and symbols indicated in FIGS. 18 to 30.

Hereinafter, with reference to FIGS. 18 to 30, the medicinal liquid supply control apparatus 80' according to the second embodiment will be described by focusing on the differences from the first embodiment described above.

The medicinal liquid supply control apparatus 80' may include a case 100, a body 200, a sealing plate 300, a reservoir 400, a reservoir pushing member 500, a button member 700, a medicinal liquid transfer tube 800, a sealer 900, a cotter 1100, at least one air passage filter 1200, and a boundary filter 1300. The medicinal liquid supply control apparatus 80' may include an elastic pushing member 1400, a support slider 1600, a slider spring 1700, a selector 1800, a lock bar 1900, a lock elastic member 2000, and a switching member 2100, and a filter spacer 2200.

FIG. 18 is a perspective view of the medicinal liquid supply control apparatus 80' according to the second embodiment of the present disclosure, illustrating ways in which cotters 1100 can be coupled to the medicinal liquid supply control apparatus 80'.

Referring to FIG. 18, the medicinal liquid supply control apparatus 80' may include a plurality of cotters 1100 provided to be suitable for respective purposes, but one cotter 1100 for medical staff of a medical team or the like may be provided and, in some cases, cotters 1100 may be used in the state of being coupled to the medicinal liquid supply control apparatus 80 at three positions (*see* 1100a, 1100b, and 1100c).

A selector hole 110h through which a selector operation part 1830 of the selector 1800 is exposed may be formed at the case 100. The selector hole 110h may be formed to be elongated to one side. The selector hole 110h may be configured to guide the movement path of the selector operation part 1830. A switching hole 110g into which the cotter 1100c is insertable to push the switching member 2100 may be formed at the case 100.

Referring to FIG. 18, the cotter 1100a may be inserted into the case 100 to prevent a user from operating the button member 700 before operating the cotter 1100a, and by pulling out the cotter 1100a in a withdrawal direction V1, the button member 700 is moved upward by the elastic pushing member 1400 and is in a state in which the user is able to push the button member 700 downward.

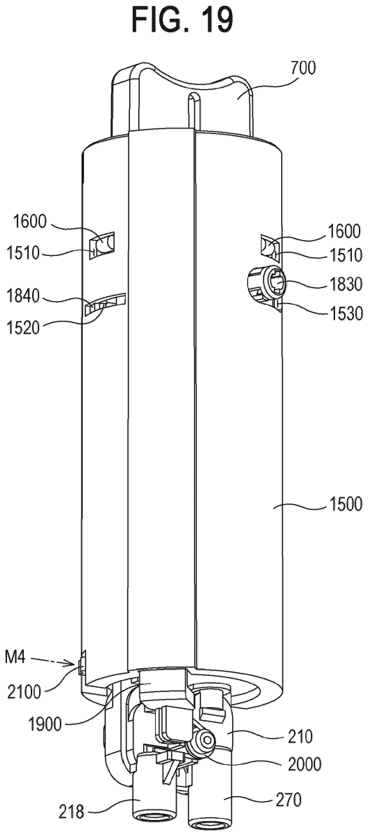

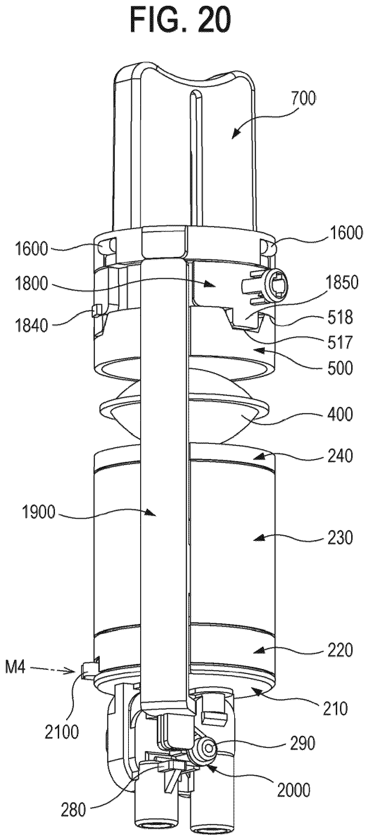

FIG. 19 is a perspective view illustrating a state in which the case 100 is removed from the medicinal liquid supply control apparatus 80' of FIG. 18. FIG. 20 is a perspective view illustrating a state in which the inner housing 1500 is additionally removed from the medicinal liquid supply control apparatus 80' of FIG. 19.

Referring to FIGS. 18 to 20, the medicinal liquid supply control apparatus 80' is configured to change the maximum inflatable volume of the reservoir 400 (hereinafter, "a medicinal liquid storage volume"). A user such as a doctor may change the liquid storage volume by coupling the cotter 1100b to the selector operation part 1830 and moving the position of the selector 1800 (*see* arrows M2 and M3). Specifically, in the state in which the selector 1800 is moved in one direction M2 and is located at a first position, the lower end surface of a step 1850 of the selector 1800 may be in contact with a first level surface517 of the reservoir pushing member 500. At this time, the upward maximum movement position of the reservoir pushing member 500 becomes relatively higher. In contrast, in the state in which the selector 1800 is moved in the other direction M3 and is located at a second position, the lower end surface of the step 1850 of the selector 1800 may be in contact with a second level surface 518 of the reservoir pushing member 500. At this time, the upward maximum movement position of the reservoir pushing member 500 becomes relatively lower.

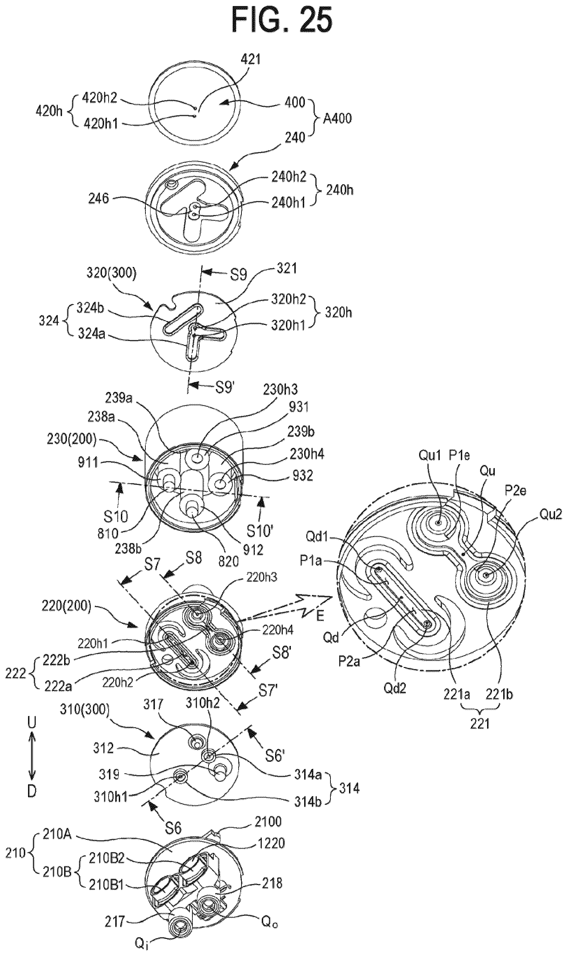

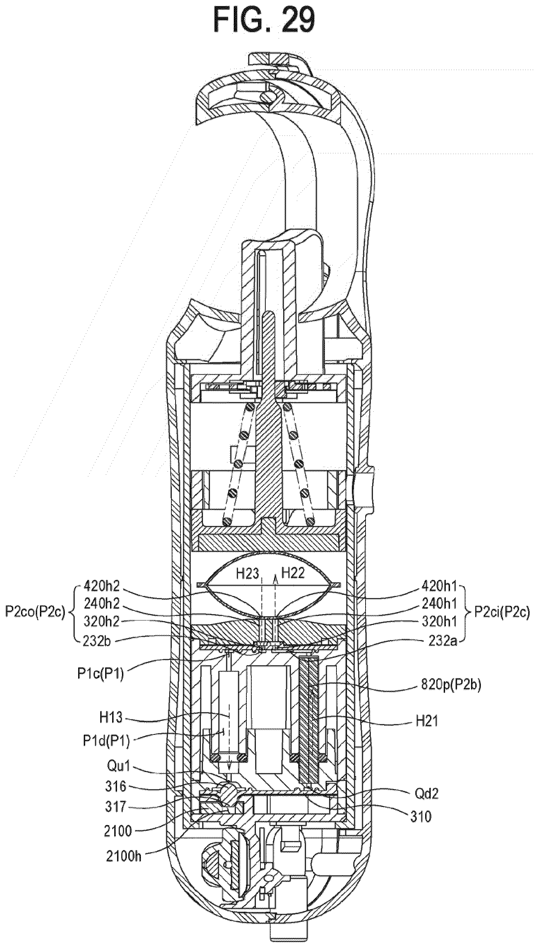

FIG. 29 is a cross-sectional view of the medicinal liquid supply control apparatus 80' taken along line S9-S9' in FIG. 25. Referring to FIGS. 18 to 20 and FIG. 29, the medicinal liquid supply control apparatus 80' may be configured to block the medicinal liquid flowing along the first channel P1 as necessary such that only the medicinal liquid passing through the second channel P2 can be injected into a patient. The medicinal liquid supply control apparatus 80' may be configured to be capable of opening/closing the first channel P1. In the initial state in which the switching member 2100 is not pushed in a preset direction M4, a first pushing portion 317 is inserted into the groove 2100h of the switching member 2100, and the first channel valve 316 opens the channel P1. By inserting the cotter 1100c into the switching hole 110g of the case 100, and pushing the switching member 2100 in the preset direction M4, the switching member 2100 is movable in the direction M4. Accordingly, the switching member 2100 pushes the first pushing portion 317 upward, the sealing plate 310 is elastically deformed, and the first channel valve 316 moves upward to close the channel P1.

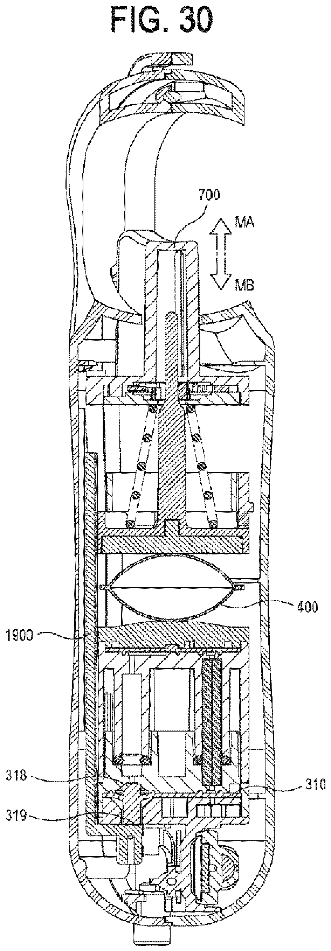

FIG. 30 is a cross-sectional view of the medicinal liquid supply control apparatus 80' taken along line S10-S10' in FIG. 25. Referring to FIGS. 18 to 20 and FIG. 30, the medicinal liquid supply control apparatus 80' may be configured to be capable of opening/closing the second channel P2. The medicinal liquid supply control apparatus 80' is configured to change the second channel P2 from the closed state to the open state when the user pushes the button member 700. In the initial state in which the button member 700 is moved upward without being pushed, the lock bar 1900 is pushed upward by the elastic restoring force of the lock elastic member 2000, and as a result, the lock bar 1900 pushes the second pushing portion 319. As the second pushing portion 319 is pushed upward, the sealing plate 310 is elastically deformed, and the second channel valve 318 moves upward to close the channel P2. In the present embodiment, the button member 700 and the lock bar 1900 are set to be spaced apart from each other in the initial state, but in another embodiment, the button member 700 and the lock bar 1900 may be set to be in contact with each other in the initial state. When the button member 700 moves downward and pushes the lock bar 1900 downward, the lock bar 1900 moves downward while elastically deforming the lock elastic member 2000 and releases the pushing on the second pushing portion 319. As the pushing on the second pushing portion 319 is released, the sealing plate 310 is elastically restored, and the second channel valve 318 moves downward to open the channel P2.

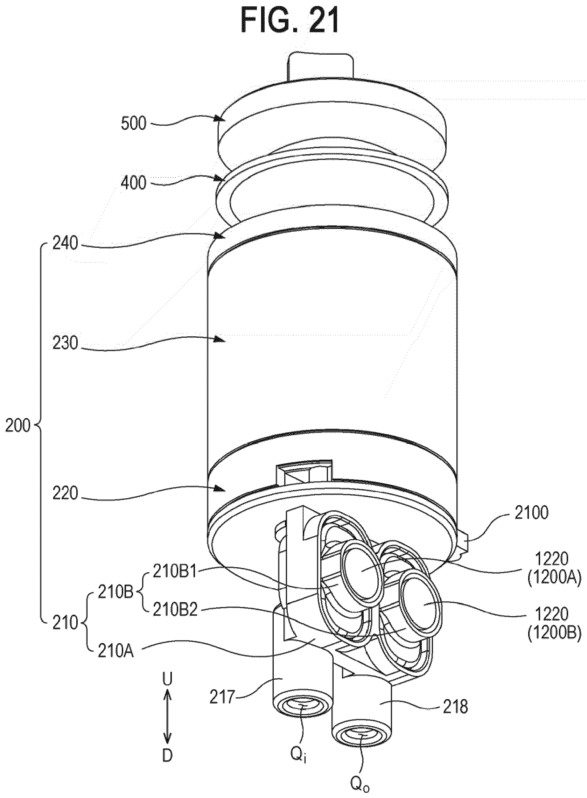

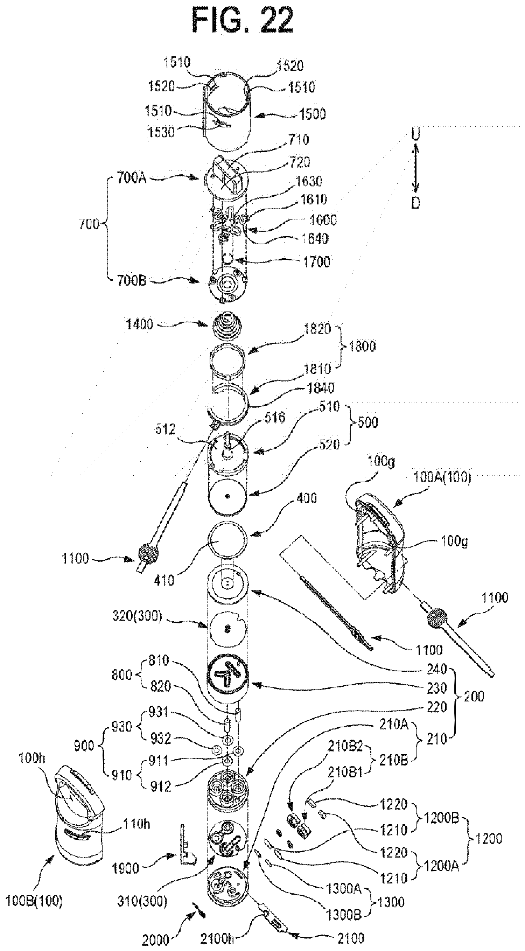

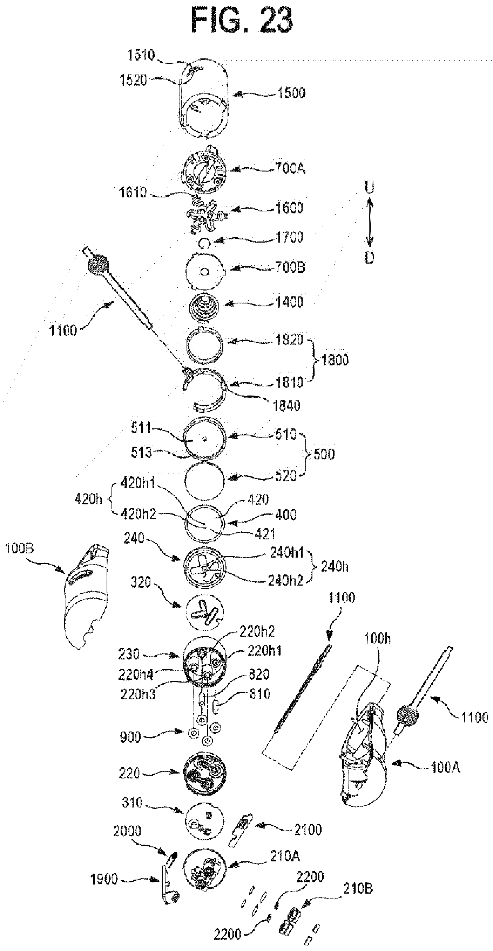

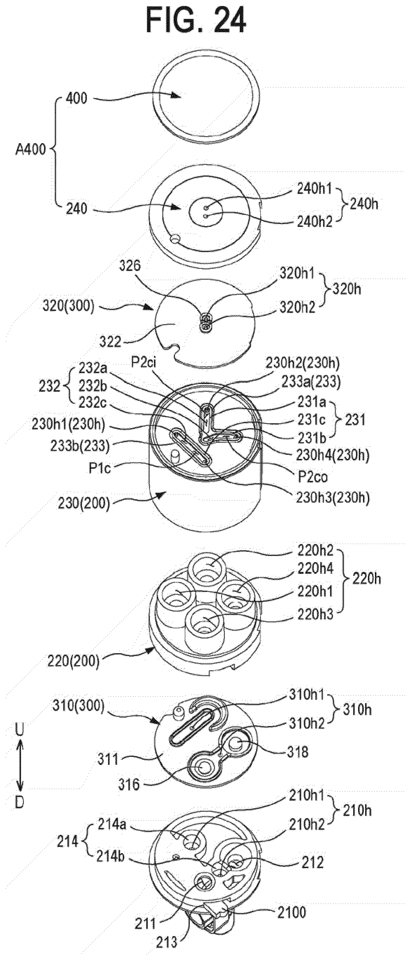

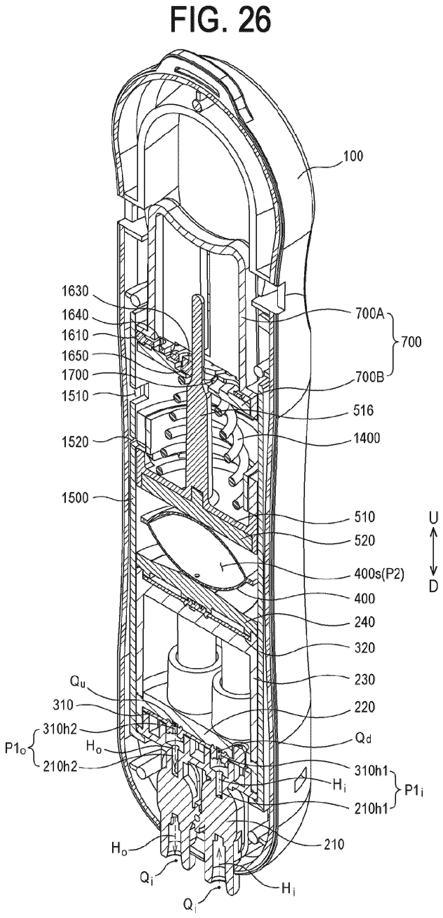

FIG. 21 is a perspective view illustrating a state in which the button member 700, the selector 1800, the lock bar 1900, and the like are additionally removed from the medicinal liquid supply control apparatus 80' of FIG. 20. FIG. 22 is an exploded perspective view of the medicinal liquid supply control apparatus 80' of FIG. 18. FIG. 23 is an exploded perspective view of the medicinal liquid supply control apparatus 80' of FIG. 22 viewed from another angle. FIG. 24 is an exploded perspective view illustrating only some of the components of FIG. 22, in which unlike FIG. 22, some components are illustrated in the state of being assembled with each other. FIG. 25 is an exploded perspective view of the state of FIG. 24 viewed from another angle, in which an enlarged view (E) of a portion is illustrated. FIG. 26 is a perspective view illustrating a cross section of the medicinal liquid supply control apparatus 80' taken along line S6-S6' in FIG. 34. Hereinafter, the configuration of the medicinal liquid supply control apparatus 80' will be described in detail with reference to FIGS. 21 to 26.

The case 100 may include an inner housing 1500. In the present embodiment, the first case part 100A, the second case part 100B, and the inner housing 1500 are coupled to each other to constitute the case 100, but in another embodiment (not illustrated), various holes corresponding to the holes formed at the inner housing 1500 may be formed in the first case part 100A and/or the second case part 100B instead, and the case 100 may be implemented without the inner housing 1500.

A first pushing portion penetration hole 211 through which the first pushing portion 317 passes and a second pushing portion penetration hole 212 through which the second pushing portion 319 passes are formed at the first part 210. A switching member arrangement hole 213 in which the switching member 2100 is arranged is formed at the first part 210. The movement path of the switching member 2100 is guided by the switching member arrangement hole 213. A portion of the cotter 1100 may be inserted into the switching member arrangement hole 213.

The upper sealing plate 320 may include a protrusion 324 protruding downward to surround the channel guide rib 231. The protrusion 324 may include a protrusion 324a surrounding the inlet channel guide 231a and the outlet channel guide 231b, and a protrusion 324b surrounding the connection channel guide 231c. The upper sealing plate 320 may include an upper protrusion 326 that protrudes upward and extends along the circumference of the connection hole 320h.

A rib groove 233 into which the protrusion 324 is inserted may be formed at the third part 230. A rib groove 233a into which the protrusion 324a is inserted and a rib groove 233b into which the protrusion 324b is inserted may be formed at the third part 230. The rib groove 233 may extend along the circumference of the channel guide rib 231. The rib groove 233 may be recessed downward.

A groove 246 into which the upper protrusion 326 is inserted may be formed at the fourth part 240. The groove 246 may be recessed upward.

An engagement groove 1510 in which an engagement protrusion 1610 of the support slider 1600 is engaged may be formed at the case 100. In the present embodiment, the engagement groove 1510 is formed at the inner housing 1500. In the description of the medicinal liquid supply control apparatus 80', a peripheral direction centered on the protrusion 516 may be defined as a circumferential direction, a direction away from the protrusion 516 may defined as a radially outward direction, and a direction approaching the protrusion 516 may be defined as a radially inward direction.

The support slider 1600 may be formed of, for example, a plastic material. The support slider 1600 may include one or more engagement protrusions 1610 protruding in the radially outward direction. The support slider 1600 may include one or more contact portions 1630 configured to come into contact with the protrusion 516 of the reservoir pushing member 500 in the radially inward direction. The support slider 1600 may include elastic connection portions 1640 configured to interconnect the engagement protrusions 1610 and the contact portions 1630 which correspond to each other and to be elastically and compressively deformable in the centrifugal direction. The support slider 1600 may include a spring engagement portion 1650 that protrudes to be engaged with the slider spring 1700.

The slider spring 1700 may be formed of, for example, a metal material. The slider spring 1700 may be coupled to the support slider 1600 to press the contact portions 1630 in the radially inward direction. The slider spring 1700 may be elastically deformed in the radially outward direction to apply an elastic restoration force to the contact portions 1630 in the radially inward direction.

The pushing surface 512 of the reservoir pushing member 500 may be pushed by the elastic pushing member 1400, rather than by the button member 700. The reservoir pushing member 500 may include a protrusion 516 protruding upward from the center.

The support slider 1600 may be disposed on the button member 700. The button member 700 and the support slider 1600 may be configured to be integrally movable upward and downward. The support slider 1600 and the slider spring 1700 may be disposed inside the button member 700. The button member 700 may include a first part 700A and a second part 700B coupled to each other.

The elastic pushing member 1400 is disposed between the button member 700 and the reservoir pushing member 500. The elastic pushing member 1400 is configured to be elastically deformed when the button member 700 is operated. The elastic pushing member 1400 may be configured to be elastically deformed when the reservoir pushing member 500 and the button member 700 come close to each other. The elastic pushing member 1400 may be configured to press the reservoir pushing member 500 downward. The elastic pushing member 1400 may use one of various known methods of exerting an elastic force. For example, the elastic pushing member 1400 may include various types of members such as a compression spring, a tension spring, a torque spring, and an air spring, or may include a member made of a material such as rubber to be elastically compressed.

The medicinal liquid supply control apparatus 80' may be configured such that a distance by which the user pushes the button member 700 is shorter than a distance by which the reservoir pushing member 500 pushes the reservoir 400. Even when the user does not push the button member 700 to the end, the elastic pushing member 1400 pushes the reservoir pushing member 500 to the end.

Although not illustrated, the medicinal liquid supply control apparatus 80' may further include an additional elastic member (not illustrated) that applies a restoring force to move the button member 700 upward. The additional elastic member is disposed between the button member 700 and the reservoir pushing member 500.

In the present embodiment, the selector 1800 includes a first part 1810 and a second part 1820 coupled to each other, but in another embodiment (not illustrated), the selector 1800 may be an integrally configured component. The selector 1800 may include a selector operation part 1830 protruding outward. The selector operation part 1830 may be exposed to the outside through the selector hole 110h, 1530. The selector 1800 may be configured such that the position in the upper-lower direction with respect to the inner housing 1500 is fixed and only the rotation with respect to the inner housing 1500 is possible. The selector 1800 may include a guide protrusion 1840 that protrudes outward and is inserted into the selector guide groove 1520 formed at the inner housing 1500 (*see* FIGS. 19 and 20). The guide protrusion 1840 may move laterally along the selector guide groove 1520, and the selector 1800 may be configured to be rotatable on the same level.

The lock bar 1900 is configured to be movable in the upper-lower direction with respect to the case 100. One end of the lock elastic member 2000 may be supported by one end of the lock bar 1900. The body 200 may include a spring support portion 280 that supports the other end of the lock elastic member 2000 and a spring arrangement portion 290 on which the lock elastic member 2000 is arranged (*see* FIG. 20).

A groove 2100h into which the first pushing portion 317 is insertable is formed at the switching member 2100. The cotter 1100 may be inserted into the apparatus through the switching hole 1540 to move the switching member 2100.

The filter spacer 2200 is disposed on the rear surface side of the air passage filter 1210. The filter spacer 2200 may be disposed to be in contact with the air passage filter 1210. The filter spacer 2200 is configured to prevent or reduce a phenomenon in which the air passage filter 1210 is bent to the downstream side by the pressure of the medicinal liquid. The filter cap 220B may be formed at an arrangement groove (not illustrated) into which the filter spacer 2200 is inserted.

The technical idea of the present disclosure has been described heretofore with reference to some embodiments and examples illustrated in the accompanying drawings. However, it is to be understood that various substitutions, modifications and alterations may be made without departing from the technical idea and scope of the present disclosure that can be understood by those of ordinary skill in the technical field to which the present disclosure pertains. In addition, it is to be understood that such substitutions, modifications, and alterations fall within the appended claims.

## Claims

1. A medicinal liquid supply control apparatus having a medicinal liquid channel that guides a flow of a medicinal liquid,
wherein the medicinal liquid channel includes a first channel that guides the medicinal liquid to flow from an inlet to an outlet, and a second channel that guides the medicinal liquid to split at a branching point of the first channel, guides the split medicinal liquid to merge at a merging point located downstream of the branching point of the first channel, and is configured to be capable of opening and closing, and
wherein the medicinal liquid supply control apparatus comprises:
a reservoir located on the second channel to define an internal space that stores the medicinal liquid, an inlet hole and an outlet hole being formed in one portion of an outer surface of the reservoir and connected to the internal space; and
a reservoir support part on which the one portion of the reservoir is fixed, an inlet connection hole constituting a portion of the second channel is connected to the inlet hole, and an outlet connection hole constituting a portion of the second channel is connected to the outlet hole being formed at the reservoir support part.

2. The medicinal liquid supply control apparatus of claim 1, wherein the reservoir support part is disposed below the reservoir, and
wherein the medicinal liquid supply control apparatus further comprises:
a reservoir pushing member disposed at an upper side of the reservoir and configured to be capable of pressing the reservoir by moving downward.

3. The medicinal liquid supply control apparatus of claim 2, wherein a gap in an upper-lower direction between an upper surface of the reservoir support part and a lower surface of the reservoir pushing member at a position corresponding to the one portion is larger than a gap in the upper-lower direction between the upper surface and the lower surface at a position different from the position corresponding to the one portion.

4. The medicinal liquid supply control apparatus of claim 2, wherein one of an upper surface of the reservoir support part and a lower surface of the reservoir pushing member includes a convex surface toward the other one, and the other one includes a concave surface corresponding to the convex surface.

5. The medicinal liquid supply control apparatus of claim 4, wherein the one portion is located at a center of a lower surface of the reservoir, and
wherein a gap in an upper-lower direction between the convex surface and the concave surface in a central portion of the reservoir is larger than a gap in the upper-lower direction between the convex surface and the concave surface in an edge portion of the reservoir.

6. The medicinal liquid supply control apparatus of claim 4, wherein the one portion is located at a center of a lower surface of the reservoir, and on a cross section crossing a center of the reservoir upward and downward, the concave surface has a larger curvature than the convex surface.

7. The medicinal liquid supply control apparatus of claim 2, wherein a lower surface of the reservoir pushing member is formed of a material that is more flexible than the reservoir support part.

8. The medicinal liquid supply control apparatus of claim 2, wherein the reservoir pushing member includes:
a reservoir pressing portion having a lower surface that presses the reservoir; and
an upper plate coupled to an upper side of the reservoir pressing portion,
wherein the reservoir pressing portion is formed of a material that is more flexible than the upper plate.

9. The medicinal liquid supply control apparatus of claim 1, further comprising:
a connection part having an upper side to which the reservoir support part is coupled; and
a sealing plate sandwiched between the reservoir support part and the connection part to be in contact with the reservoir support part and the connection part, a first connection hole constituting a portion of the second channel is connected to the inlet connection hole, and a second connection hole constituting a portion of the second channel is connected to the outlet connection hole being formed at the sealing plate.

10. The medicinal liquid supply control apparatus of claim 1, wherein the reservoir is configured to change a volume of the internal space.

11. A reservoir assembly for a medicinal liquid supply control apparatus having a medicinal liquid channel that guides a flow of a medicinal liquid, the reservoir assembly comprising:
a reservoir located on the medicinal liquid channel to define an internal space that stores the medicinal liquid, an inlet hole and an outlet hole being formed in one portion of an outer surface of the reservoir and connected to the internal space; and
a reservoir support part on which the one portion of the reservoir is fixed, an inlet connection hole constituting a portion of the medicinal liquid channel is connected to the inlet hole, and an outlet connection hole constituting a portion of the medicinal liquid channel is connected to the outlet hole being formed at the reservoir support part.

12. The reservoir assembly of claim 11, wherein an upper surface of the reservoir support part includes an upwardly convex surface.

13. The reservoir assembly of claim 12, wherein the one portion is located at a center of a lower surface of the reservoir, and the convex surface is provided to protrude from a position corresponding to the center.

14. The reservoir assembly of claim 11, wherein the inlet hole and the inlet connection hole are vertically connected to each other, and
wherein the outlet hole and the outlet connection hole are vertically connected to each other.

15. A medicinal liquid injection apparatus comprising:
a pumping module configured to press a medicinal liquid;
an extension tube configured to allow the medicinal liquid flowing out from the pumping module by being pressed in the pumping module to flow therethrough; and
a medicinal liquid supply control apparatus having a medicinal liquid channel connected to the extension tube,
wherein the medicinal liquid channel includes a first channel that guides the medicinal liquid to flow from an inlet to an outlet, and a second channel that guides the medicinal liquid to split at a branching point of the first channel, guides the split medicinal liquid to merge at a merging point located downstream of the branching point of the first channel, and is configured to be capable of opening and closing, and
wherein the medicinal liquid supply control apparatus comprises:
a reservoir located on the second channel to define an internal space that stores the medicinal liquid, an inlet hole and an outlet hole being formed in one portion of an outer surface of the reservoir and connected to the internal space; and
a reservoir support part on which the one portion of the reservoir is fixed, an inlet connection hole constituting a portion of the second channel is connected to the inlet hole, and an outlet connection hole constituting a portion of the second channel is connected to the outlet hole being formed at the reservoir support part.
